# EUROPEAN PATENT SPECIFICATION

(11) **EP 1 219 173 B1**
(45) Date of publication and mention of the grant of the patent: **16.06.2004**
(21) Application number: 00961164.1
(22) Date of filing: 21.09.2000
(51) Int. Cl.: A01N 43/56, C07D 231/12, C07D 231/18, C07D 401/12, C07D 403/12, C07D 417/12, C07D 405/12, C07D 409/12, C07D 403/06

(54) **INSECTICIDES AND ACARICIDES**
INSEKTIZIDE UND AKARIZIDE
INSECTICIDES ET ACARICIDES

(30) Priority: 24.09.1999 JP 27086199
(43) Date of publication of application: 03.07.2002
(73) Proprietor: Agro-Kanesho Co., Ltd., Tokyo 107-0052 (JP)
(72) Inventor: ODA, Masatsugu, Mitsubishi Chemical Corporation, Yokohama-shi, Kanagawa 227-0033 (JP); KATSURADA, Manabu, Mitsubishi Chemical Corporation, Yokohama-shi, Kanagawa 227-0033 (JP); SHIGA, Yasushi, Mitsubishi Chemical Corporation, Yokohama-shi, Kanagawa 227-0033 (JP); FUKUCHI, Toshiki, Mitsubishi Chemical Corporation, Yokohama-shi, Kanagawa 227-0033 (JP); KATO, Taku, Mitsubishi Chemical Corporation, Yokohama-shi, Kanagawa 227-0033 (JP)
(74) Representative: VOSSIUS & PARTNER
(86) International application number: PCT/JP2000/006479
(87) International publication number: WO 2001/020993

(56) References cited:
- EP-A1- 0 295 117
- EP-A1- 0 433 899
- EP-A1- 0 571 326
- EP-A1- 0 658 547
- GB-A- 2 046 734
- JP-A- 3 093 774
- JP-A- 57 040 467
- PATENT ABSTRACTS OF JAPAN vol. 017, no. 644 (C-1134), 30 November 1993 (1993-11-30) & JP 05 201980 A (MITSUBISHI KASEI CORP), 10 August 1993 (1993-08-10)
- PATENT ABSTRACTS OF JAPAN vol. 1995, no. 11, 26 December 1995 (1995-12-26) & JP 07 224041 A (MITSUBISHI CHEM CORP), 22 August 1995 (1995-08-22)

## Description

The present invention relates to the use of a pyrazolyl derivative as an insecticide and acaricide.

Various insecticides were developed and practically used for the purpose of preventing and exterminating various vermin in the agricultural and horticultural fields. Insecticides recently developed are, for example, pyrazolylamide compounds.

However, insecticidal and acaricidal agents have a serious problem that vermin which became resistant to ordinary chemicals appeared to make the control of them difficult. For this reason, the development of new insecticidal and acaricidal agents is always demanded. In addition, recently the demand of insecticidal and acaricidal agents harmless to living bodies other than the target vermin and also to the environment is increasing more and more. Thus, there is strong demand in development of new insecticidal and acaricidal agents which are more excellent than the ordinary ones in the insecticidal and acaricidal effects, insecticidal and acaricidal spectrum, safety and environmental problems.

On the other hand, it is known that pyrazolyl acrylate compounds and pyrazolyl carbamate compounds have a fungicidal effect. For example, EP 433 899 discloses the following compound:

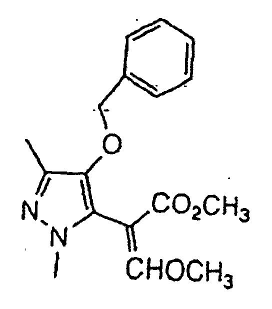

EP 571 326 discloses the following compound:

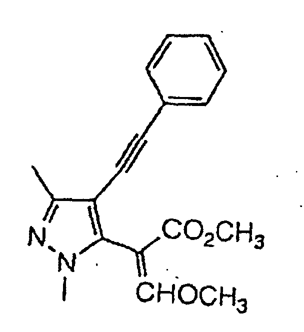

Japanese Patent Unexamined Published Application (hereinafter referred to as "J. P. KOKAI") No. Hei 5-201980 discloses the following compound:

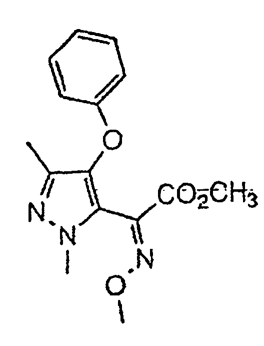

J. P. KOKAI No. Hei 7-224041 discloses the following compound:

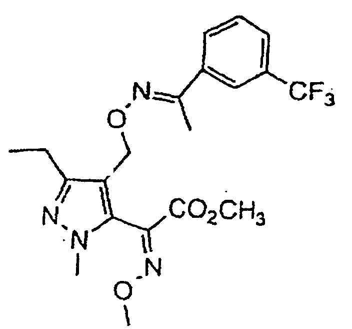

EP 658 547 discloses the following compound:

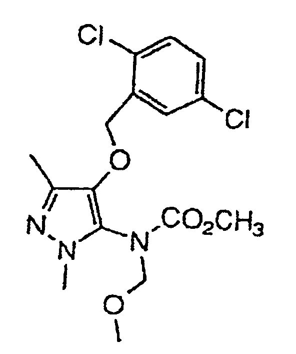

However, these Official Gazettes disclose only the effects of those compounds as the agricultural and horticultural fungicides, but they are completely silent on the physiological activities, namely, insecticidal and acaricidal activities. It is not so common in this technical field that compounds practically usable as fungicides also have practical insecticidal and acaricidal activities.

EP-A-0 295 117 describes certain derivatives of N-phenylpyrazoles which are stated to possess arthropodicidal, plant nematocidal, anthelmintic and anti-protozoal properties.

The object of the present invention is to provide a highly safe insecticide and acaricide having a high effect of controlling various vermin resistant to ordinary agricultural and horticultural insecticides and acaricides, and also of reducing problems of residual toxicity and environmental pollution to a considerable extent.

After intensive investigations made for the purpose of solving the above-described problems, the inventors have found that pyrazolyl compounds having a specific structure have not only the fungicidal effect but also excellent insecticidal and acaricidal activities. The present invention has been completed on the basis of this finding. Namely, the present invention relates to the use of a pyrazolyl derivative of the following general formula (I): 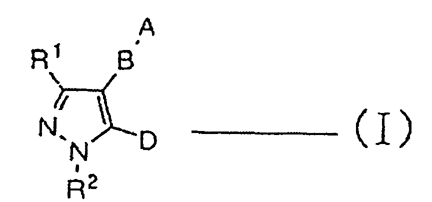 wherein
A represents a hydrogen atom; an alkyl group which may be substituted; an alkenyl group which may be substituted; an alkynyl group which may be substituted; a tri-substituted silyl group substituted with an alkyl group and/or an aryl group; an aryl group which may be substituted; or a heterocyclic group which may be substituted;
B represents a single bond; a group of the formula: -(G¹)ₙ-G²-(G¹)ₘ- wherein G¹ represents an oxygen atom, a sulfur atom, a sulfinyl group or a sulfonyl group, G² represents an alkylene group, an alkenylene group or an alkynylene group, and n and m are independent from each other and represent 0 or 1; carbonyl group; a group of the formula: -CH₂-O-N=C(R³)- wherein R³ represents a hydrogen atom, an alkyl group or a haloalkyl group; or a group of the formula: -CH=N-O-(CR³R⁴)ₙ- wherein R³ and R⁴ each represents a hydrogen atom, an alkyl group or a haloalkyl group; and n is 0 or 1,
R¹ represents a hydrogen atom; a halogen atom, an alkyl group which may be substituted; an alkenyl group which may be substituted; an alkynyl group which may be substituted; an alkoxyl group which may be substituted; or an aryl group which may be substituted,
R² represents a hydrogen atom; an alkyl group; a haloalkyl group; or an aryl group which may be substituted, and
D represents a group of the formula: -C(=Y)COX wherein X represents a hydroxyl group, an alkoxyl group or an alkylamino group, Y represents a group of the formula: CH-(G³)ₙ-G⁴ wherein G³ represents an oxygen atom or a sulfur atom, G⁴ represents an alkyl group or a haloalkyl group, and n represents 0 or 1, a group of the formula: N-O-G⁴ wherein G⁴ represents an alkyl group or a haloalkyl group; or a group of the formula: -N(R⁵)CO₂G⁵ wherein R⁵ represents an alkyl group, an alkenyl group, an alkynyl group, an alkylthioalkyl group or an alkoxyalkyl group, and G⁵ represents an alkyl group; as an insecticide or acaricide.

The detailed description will be made on the present invention.

The pyrazolyl derivatives used as an insecticide and acaricide according to the present invention are represented by the above general formula (I).

In the general formula (I), A represents a hydrogen atom; a linear, branched or cyclic alkyl group, which may be substituted, such as methyl group, ethyl group, n-propyl group, iso-propyl group, n-butyl group, sec-butyl group, n-pentyl group, n-hexyl group, cyclopropyl group, cyclobutyl group or cyclohexyl group; a linear, branched or cyclic alkenyl group, which may be substituted, such as vinyl group, propenyl group, butenyl group or hexenyl group; a linear or branched or cyclic alkynyl group, which may be substituted, such as ethynyl group, butynyl group or pentynyl group; a tri-substituted silyl group, which is substituted with an alkyl group and/or an aryl group, such as trimethylsilyl group, triethylsilyl group or diphenylmethylsilyl group; an aryl group, which may be substituted, such as phenyl group or naphthyl group; or a heterocyclic group, which may be substituted, such as pyridyl group, pyrimidyl group, thiazolyl group, benzothiazolyl group, oxazolyl group, benzoxazolyl group, furyl group, thienyl group, morpholinyl group, benzodioxanyl group or benzofuranyl group.

The above-described alkyl groups, alkenyl groups and alkynyl groups are preferably those lower ones having 10 or less carbon atoms, and the tri-substituted silyl groups are preferably those having 12 or less carbon atoms.

The substituents of the alkyl groups include halogen atoms such as fluorine atom, chlorine atom and bromine atom; C₁-C₄ alkoxyl groups such as methoxyl group, ethoxyl group, iso-propoxyl group and n-butoxyl group; and aryl groups such as phenyl group. The halogen atoms and alkoxyl groups are preferred.

The substituents of the alkenyl groups and alkynyl groups include halogen atoms such as fluorine atom, chlorine atom and bromine atom; C₁-C₄ alkyl groups such as methyl group, ethyl group, n-propyl group, iso-propyl group, n-butyl group and sec-butyl group; and C₁-C₄ alkoxyl groups such as methoxyl group, ethoxyl group, iso-propoxyl group and n-butoxyl group.

The aryl groups and heterocyclic groups are preferably phenyl group, pyrimidyl group, thiazolyl group and thienyl group, which may be substituted.

The substituents of the aryl groups and heterocyclic groups include halogen atoms such as fluorine atom, chlorine atom and bromine atom; C₁-C₆ alkyl groups such as methyl group, ethyl group, n-propyl group, iso-propyl group, n-butyl group, sec-butyl group and cyclohexyl group; C₁-C₆ haloalkyl groups such as trifluoromethyl group, difluoromethyl group; trichloromethyl group and dichlorodifluoroethyl group; C₁-C₆ alkoxyl groups such as methoxyl group, ethoxyl group, iso-propoxyl group and n-butoxyl group; C₁-C₆ haloalkoxyl groups such as difluoromethoxyl group, trifluoromethoxyl group, trifluoroethoxyl group and 1-trifluoromethylethoxyl group; C₁-C₆ alkylthio groups such as methylthio group, ethylthio group, n-propylthio group and sec-butylthio group; aryl groups such as phenyl group and naphthyl group; aryloxy groups such as phenoxy group; and heteroaryloxy groups such as pyridyloxy group. The aryl groups, aryloxy groups and heteroaryloxy groups may be further substituted with a substituent selected from the group consisting of halogen atoms, C₁-C₆ alkyl groups, C₁-C₆ haloalkyl groups, C₁-C₆ alkoxyl groups, C₁-C₆ haloalkoxyl groups and C₁-C₆ alkylthio groups. As for the substituents of the aryl groups and heterocyclic groups, two substituents adjacent to each other may together form methylenedioxy group, ethylenedioxy group or the like to form a condensed ring with the aryl group or heterocyclic group. The number of the substituents is 1 to 5, preferably 1 to 3. When they have two or more substituents, the substituents may be the same or different from each other. The substituents of the aryl groups and heterocyclic groups are preferably halogen atoms; alkyl groups, haloalkyl groups, alkoxyl groups, haloalkoxyl groups, phenoxyl group; and pyridyloxy group (the phenoxyl group and pyridyloxy group may be substituted with a substituent selected from the group consisting of halogen atoms, alkyl groups, haloalkyl groups, alkoxyl groups, haloalkoxyl groups and alkylthio groups).

A is preferably an aryl group or heterocyclic group which may be substituted with a substituent selected from the group consisting of halogen atoms, alkyl groups, haloalkyl groups, alkoxyl groups, haloalkoxyl groups, alkylthio groups, aryl groups which may be substituted, aryloxy groups which may be substituted, and heteroaryloxy groups which may be substituted (the substituents of the aryl groups, aryloxy groups and heteroaryloxy groups are selected from the group consisting of halogen atoms, alkyl groups, haloalkyl groups, alkoxyl groups, haloalkoxyl groups and alkylthio groups). A is more preferably a substituted phenyl group having a substituent at, at least the 4-position, or a substituted phenyl group having substituents at, at least the 3- and 5-positions independently, the substituents being selected from a group consisting of halogen atoms, alkyl groups, haloalkyl group, alkoxyl groups, haloalkoxyl groups, alkylthio groups, aryl groups which may be substituted, aryloxy groups which may be substituted and heteroaryloxy groups (the substituents of the aryl groups, aryloxy groups and heteroaryloxy groups are selected from the group consisting of halogen atoms, alkyl groups, haloalkyl groups, alkoxyl groups, haloalkoxyl groups and alkylthio groups). A is most preferably a disubstituted phenyl group having substituents at the 3- and 5-positions, the substituents being selected from the group consisting of halogen atoms, C₁-C₂ haloalkyl groups and C₁-C₄ haloalkoxyl groups; or a monosubstituted phenyl group having a substituent at the 4-position, the substituents being selected from a group consisting of halogen atoms, C₁-C₂ haloalkyl groups, C₁-C₄ haloalkoxyl groups, phenoxyl group and pyridyloxy group (the phenoxyl group and pyridyloxy group may be substituted with a substituent selected from the group consisting of halogen atoms, alkyl groups, haloalkyl groups, alkoxyl groups, haloalkoxyl groups and alkylthio groups).

B represents a single bond; a group of the formula: -(G¹)ₙ-G²-(G¹)ₘ- wherein G¹ represents an oxygen atom, sulfur atom, sulfinyl group or sulfonyl group, G² represents an alkylene group, an alkenylene group or an alkynylene group, and n and m are independent from each other and represent 0 or 1; carbonyl group; a group of the formula: -CH₂-O-N=C(R³)- wherein R³ represents a hydrogen atom, an alkyl group or a haloalkyl group; or a group of the formula: -CH=N-O-(CR³R⁴)ₙ- wherein R³ and R⁴ each represents a hydrogen atom, an alkyl group or a haloalkyl group, and n is 0 or 1. The alkylene groups, alkenylene groups and alkynylene groups are preferably lower ones having 4 or less carbon atoms, particularly those having 2 or less carbon atoms.

B is preferably a single bond; -OCH₂-, -CH₂O-, -CH₂S-, -CH₂SO-, -CH₂SO₂-, -C≡C-; -CH=CH-; -CH₂CH₂-; -CO-; a group of the formula: -CH₂ON=C(R³)- or a group of the formula: -CH=NO-(CR³R⁴)ₙ-. B is more preferably -OCH₂-; -CH₂O-; -C≡C-; -CH=CH-; - CH₂CH₂-; a group of the formula: -CH₂ON=C(R³)- or a group of the formula: -CH=NO-(CR³R⁴)ₙ-. B is particularly preferably -OCH₂-; -C≡C-; -CH=CH- or -CH₂CH₂-. B is most preferably -OCH₂- or C≡C-.

R³ and R⁴ each represent a hydrogen atom, an alkyl group such as methyl group, ethyl group, n-propyl group, iso-propyl group, n-butyl group or sec-butyl group; or a haloalkyl group such as trifluoromethyl group, difluoromethyl group, trichloromethyl group or dichlorodifluoroethyl group. The alkyl groups and haloalkyl groups are preferably lower ones having 4 or less carbon atoms.

R³ and R⁴ are each preferably a hydrogen atom or a methyl group and n is 0 or 1. n is preferably 1.

R¹ represents a hydrogen atom; a halogen atom such as fluorine atom, chlorine atom or bromine atom, a linear, branched or cyclic alkyl group, which may be substituted, such as methyl group, ethyl group, n-propyl group, iso-propyl group, n-butyl group, sec-butyl group, cyclopropyl group, cyclobutyl group or cyclohexyl group; a linear, branched or cyclic alkenyl group, which may be substituted, such as vinyl group, propenyl group, butenyl group or hexenyl group; a linear or branched alkynyl group, which may be substituted, such as ethynyl group, butynyl group or pentynyl group; a linear, branched or cyclic alkyloxy group, which may be substituted, such as methoxyl group, ethoxyl group, iso-propoxyl group or n-butoxyl group; or an aryl group, which may be substituted, such as phenyl group or naphthyl group.

The alkyl groups, alkenyl groups, alkynyl groups and alkoxyl groups are preferably lower ones having 10 or less carbon atoms.

The substituents of the alkyl groups include halogen atoms such as fluorine atom, chlorine atom and bromine atom; and C₁-C₄ alkoxyl groups such as methoxyl group, ethoxyl group, iso-propoxyl group and n-butoxyl group. The halogen atoms are preferred.

The substituents of the alkenyl groups, alkynyl groups and alkoxyl groups include halogen atoms such as fluorine atom, chlorine atom and bromine atom; C₁-C₄ alkyl groups such as methyl group, ethyl group, n-propyl group, iso-propyl group, n-butyl group and sec-butyl group; and C₁-C₄ alkoxyl groups such as methoxyl group, ethoxyl group, iso-propoxyl group and n-butoxyl group.

The substituents of the aryl groups include halogen atoms such as fluorine atom, chlorine atom and bromine atom; C₁-C₆ alkyl groups such as methyl group, ethyl group, n-propyl group, iso-propyl group, n-butyl group, sec-butyl group and cyclohexyl group; C₁-C₆ haloalkyl groups such as trifluoromethyl group, difluoromethyl group, trichloromethyl group and dichlorodifluoroethyl group; C₁-C₆ alkoxyl groups such as methoxyl group, ethoxyl group, iso-propoxyl group and n-butoxyl group; and C₁-C₆ haloalkoxyl groups such as difluoromethoxyl group, trifluoromethoxyl group, trifluoroethoxyl group and 1-trifluoromethylethoxyl group.

R¹ is preferably a hydrogen atom; a halogen atom; an alkyl group; a haloalkyl group; an alkoxyl group; a haloalkoxyl group or an aryl which may be substituted. R¹ is particularly preferably a hydrogen atom; a halogen atom; an alkyl group; a haloalkyl group; an alkoxyl group; a haloalkoxyl group; or an aryl which may be substituted with a substituent selected from the group consisting of halogen atoms, alkyl groups, haloalkyl groups, alkoxyl groups and haloalkoxyl groups. R¹ is most preferably a hydrogen atom, a C₁-C₄ alkyl group, trifluoromethyl group or phenyl group.

R² represents a hydrogen atom; an alkyl group such as methyl group, ethyl group, n-propyl group, iso-propyl group, n-butyl group or sec-butyl group; a haloalkyl group such as trifluoromethyl group, difluoromethyl group, trichloromethyl group or dichlorodifluoroethyl group; or an aryl group, which may be substituted, such as phenyl group or naphthyl group.

The alkyl groups and haloalkyl groups are preferably lower ones having 6 or less carbon atoms. The substituents of the aryl groups are the same as those listed above for R¹.

R² is preferably an alkyl group; a haloalkyl group; or an aryl group which may be substituted with a substituent selected from the group consisting of halogen atoms, alkyl groups, haloalkyl groups, alkoxyl groups and haloalkoxyl groups. R² is particularly preferably a C₁-C₄ alkyl group, a C₁-C₄ haloalkyl group or phenyl group.

D represents a group of the formula: -C(=Y)COX or a group of the formula: -N(R⁵)CO₂G⁵.

X represents a hydroxyl group; an alkoxyl group such as methoxyl group, ethoxyl group, iso-propoxyl group or n-butoxyl group; or an alkylamino group such as methylamino group or ethylamino group. The alkoxyl groups and alkylamino groups are those having 6 or less carbon atoms, preferably 2 or less carbon atoms. X is preferably a methoxyl group.

Y represents a group of the formula: CH-(G³)ₙ-G⁴ wherein G³ represents an oxygen atom or a sulfur atom, G⁴ represents an alkyl group or a haloalkyl group, and n represents 0 or 1; or a group of the formula: N-O-G⁴ wherein G⁴ represents an alkyl group or a haloalkyl group. Y is preferably CHOCH₃, CHCH₃, CHC₂H₅, CHSCH₃ or NOCH₃. Y is more preferably CHOCH₃, CHCH₃ or CHC₂H₅. Y is particularly preferably CHOCH₃.

R⁵ represents an alkyl group such as methyl group, ethyl group, n-propyl group, iso-propyl group, n-butyl group or sec-butyl group; an alkenyl group such as vinyl group, propenyl group or butenyl group; an alkynyl group such as propargyl group; an alkylthioalkyl group such as methylthiomethyl group or ethylthiomethyl group; or an alkoxyalkyl group such as methoxymethyl group or ethoxyethyl group. The alkyl groups, alkenyl groups, alkynyl groups, alkylthioalkyl groups and alkoxyalkyl groups are those having 4 or less carbon atoms.

R⁵ is preferably ethyl group, n-propyl group, propargyl group or methoxymethyl group.

G⁵ is an alkyl group such as methyl group or ethyl group. G⁵ is preferably a methyl group.

When D in the pyrazolyl derivative of the general formula (I) is a group represented by the formula: -C(=Y)COX, there are geometrical isomers (E / Z) due to the C=Y double bond. According to the present invention both isomers are usable as an insecticide and acaricide.

Although most of the compounds represented by the general formula (I) are included by those of general formulae given in EP 433 899, EP 571 326, J. P. KOKAI No. Hei 5- 201980, J. P. KOKAI No. Hei 7-224041 and EP 658 547, these known publications are utterly silent about the insecticidal activity and acaricidal activity of them. The present invention provides such a new use of them.

Further, pyrazolylacrylic acid derivatives of the following general formula (II) have the most excellent insecticidal and acaricidal effect:

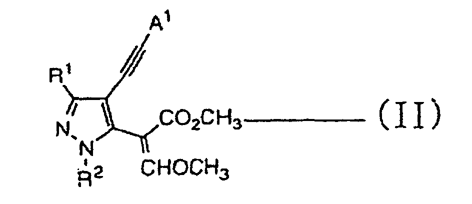

In the general formula (II), A¹ represents a phenyl group substituted at, at least, the 4-position or a phenyl group substituted at 3- and 5-positions independently from each other, the substituents being selected from the group consisting of halogen atoms, alkyl groups, haloalkyl groups, alkoxyl groups, haloalkoxyl groups, alkylthio groups, aryl groups which may be substituted, aryloxy groups which may be substituted and heteroaryloxy groups which may be substituted (the substituents of the aryl groups, aryloxy groups and heteroaryloxy groups are selected from the group consisting of halogen atoms, alkyl groups, haloalkyl groups, alkoxyl groups, haloalkoxyl groups and alkylthio groups).

The alkyl groups, haloalkyl groups, alkoxyl groups, haloalkoxyl groups and alkylthio groups are preferably lower ones having 6 or less carbon atoms.

R¹ and R² are as defined above in the general formula (I).

The following compounds are excluded from those of the above general formula (II): methyl α-{1,3-dimethyl-4-(4-chlorophenylethynyl)-5-pyrazole}-β-methoxyacrylate; methyl α-{1,3-dimethyl-4-(4-fluorophenylethynyl)-5-pyrazole}-β-methoxyacrylate; methyl α-{1,3-dimethyl-4-(4-methoxylphenylethynyl)-5-pyrazole}-β-methoxyacrylate; methyl α-{1,3-dimethyl-4-(4-methylphenylethynyl)-5-pyrazole}-β-methoxyacrylate; methyl a -{1,3-dimethyl-4-(3,4-dichlorophenylethynyl)-5-pyrazole}-β-methoxyacrylate; methyl α-{1,3-dimethyl-4-(3-chloro-4-methoxyphenylethynyl)-5-pyrazole}-β-methoxyacrylate; methyl α -{1,3-dimethyl-4-(2,4-dichlorophenylethynyl)-5-pyrazole}-β-methoxyacrylate; methyl α-{1-methyl-3-trifluoromethyl-4-(4-chlorophenylethynyl)-5-pyrazole}-β-methoxyacrylate; methyl α-{1-methyl-3-trifluoromethyl-4-(4-fluorophenylethynyl)-5-pyrazole}-β-methoxyacrylate; methyl α-{1-methyl-3-trifluoromethyl-4-(3-chloro-4-methoxyphenylethynyl)-5-pyrazole}-β-methoxyacrylate; and methyl α-{1-methyl-3-trifluoromethyl-4-(2,4-dichlorophenplethynyl)-5-pyrazole}-β-methoxyacrylate.

The pyrazolyl derivatives represented by the general formula (I) can be produced by the processes disclosed in EP 433 899, EP 571 326, J. P. KOKAI No. Hei 5- 201980, J. P. KOKAI No. Hei 7-224041 and EP 658 547 or those processes based on them.

The above-described new compounds (II) of the present invention can also be produced by the processes disclosed in those publications. Further, the following process is economically advantageous because the number of the steps is only small and also the starting materials used are inexpensive: 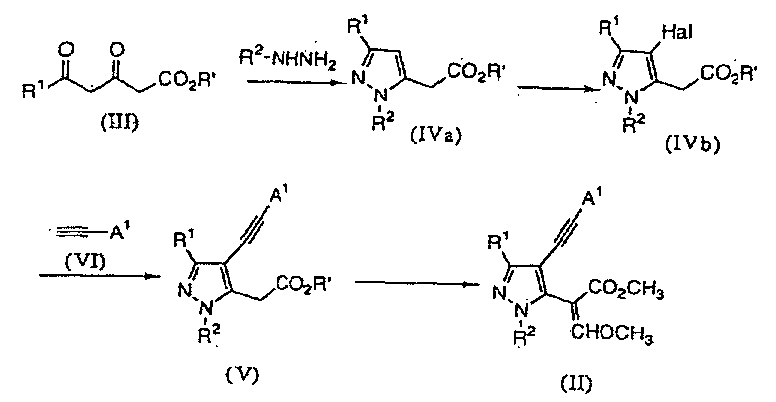 wherein R' represents an alkyl group, Hal represents a halogen atom, and A¹, R¹ and R² are as defined in the above general formula (I).

In the above reaction scheme, R' represents an alkyl group preferably having 6 or less carbon atoms, such as methyl group, ethyl group, n-propyl group, iso-propyl group, sec-butyl group or tert-butyl group. R' is particularly preferably methyl group, iso-propyl group or tert-butyl group. Hal represents a halogen atom such as fluorine atom, chlorine atom, bromine atom or iodine atom. HaI is preferably a iodine atom.

Pyrazolylacetic acid derivatives represented by the general formulae (IVa) and (IVb) are intermediates formed by the above-described synthesis reactions.

The pyrazolylacrylic acid derivatives represented by the general formula (II) are obtained by methylating β-hydroxypropenic acid ester (or a salt thereof) obtained by reacting a pyrazolylacetic acid derivative (V) with methyl formate in the presence of a base (Claisen reaction).

Examples of the bases used for Claisen reaction as described above are alkali metal hydrides such as sodium hydride; alkali metal alcoholates such as sodium methylate; alkali metal carbonates such as potassium carbonate; alkali metal hydroxides such as potassium hydroxide; and tertiary amines such as N-methylmorpholine and triethylamine; and aromatic bases such as pyridine and picoline.

The bases used for the methylation reaction are also selected from the group consisting of the above-described examples of the bases, and they may be the same or different from those used for Claisen reaction.

The methylating agents are, for example, methyl iodide and dimethyl sulfate.

The solvents used for Claisen reaction and methylation reaction are, for example, aromatic hydrocarbons such as benzene, toluene and xylene; halogenated hydrocarbons such as dichloromethane, chloroform and 1,2-dichloroethane; ethers such as diethyl ether, tetrahydrofuran and dioxane; esters such as ethyl acetate; alcohols such as methanol, ethanol and propanol; and polar solvents such as N,N-dimethylformamide, N-methylpyrrolidone, dimethyl sulfoxide and acetonitrile. They may be used alone or in the form of a mixture of them. In those solvents, the polar solvents such as N,N-dimethylformamide and N-methylpyrrolidone are preferred.

In a preferred mode of the reaction, the base is added at a temperature of -10°C to 50°C in the reaction with methyl formate, the reaction is carried out at 0 to 100°C for 2 to 24 hours and after the completion of the reaction, the methylating agent is added at a temperature of -10°C to 50°C and the reaction is carried out at 0 to 100°C for 1 to 24 hours to complete the methylation.

Compounds (I) obtained by this reaction have geometrical isomers (E / Z) due to the methoxy acrylate fraction. Although both isomers are included in the present patent, E-isomer is preferred from the viewpoint of the insecticidal and acaricidal effects.

. The isomers can be divided from each other by a method usually employed for separating the geometrical isomers from each other, such as the chromatography.

The pyrazolylacetic acid derivatives (V) can be obtained by reacting a corresponding halogen derivative (IVb) with an ethynyl derivative in the presence of a base and a palladium catalyst in a solvent inert to the reaction.

The bases usable for the reaction include amines such as diethylamine, butylamine and triethylamine; aromatic bases such as pyridine and picoline; and inorganic salts such as potassium carbonate and sodium hydrogencarbonate. Preferred bases include diethylamine and triethylamine. The base is used in an amount ranging from 0.1 equivalent per 1 equivalent of the halogen derivative (IVb) to a highly excess amount.

The solvents used are, for example, aromatic hydrocarbons such as benzene, toluene and xylene; halogenated hydrocarbons such as dichloromethane, chloroform and 1,2-dichloroethane; ethers such as diethyl ether, tetrahydrofuran, 1,2-dimethoxyethane and dioxane; esters such as ethyl acetate; and polar solvents such as N,N-dimethylformamide, N-methylpyrrolidone, dimethyl sulfoxide and acetonitrile. They can be used either alone or in the form of a solvent mixture. In those solvents, the polar solvents such as N,N-dimethylformamide and N-methylpyrrolidone are preferred. When the above-described base is used in a highly excess amount, the reaction can proceed without using the solvent because the base per se acts also as the solvent.

The catalysts used for the reaction are, for example, tetrakistriphenylphosphine palladium (0), dichloroditriphenylphosphine palladium (II), diacetoxyditriphenylphosphine palladium (II) and palladium carbon. The reaction smoothly proceeds in the presence of any of these catalysts.

The catalyst is used in an amount of 0.001 to 1 equivalent, preferably 0.005 to 0.2 equivalent, per equivalent of the halogen derivative (IVb).

The reaction is further accelerated in the copresence of 0.001 to 1 equivalent, preferably 0.005 to 0.5 equivalent, of a copper salt such as copper iodide per equivalent of the halogen derivative (IVb).

The ethynyl derivative is used for the reaction in an amount of 0.5 to 10 equivalents, preferably 1 to 3 equivalents, per equivalent of the halogen derivative (IV). The reaction is carried out at 0 to 150°C, preferably 10 to 100°C.

The halogen derivatives (IVb) can be obtained by treating a corresponding pyrazolylacetic acid derivative (IVa) with a halogenating agent such as chlorine, bromine, iodine, N-bromosuccinimide or sulfuryl chloride in the presence of a catalyst such as periodic acid, perbenzoic acid or 2,2'-azobis(isobutyronitrile) or under the irradiation with a light in a solvent inert to the reaction.

The solvents usable for the reaction are halogenated hydrocarbons such as carbon tetrachloride; aromatic hydrocarbons such as chlorobenzene; and polar solvents such as acetic acid and water.

For the reaction, 0.5 to 1.5 equivalents of the halogenating agent is used per equivalent of the pyrazolylacetic acid derivative (IVa), and the reaction is carried out usually at 0 to 150°C, preferably at 10 to 100°C, for 1 to 6 hours.

The pyrazolylacetic acid derivatives (IVa) can be obtained by reacting a dioxocarboxylic acid ester (III) with a hydrazine derivative or salt thereof in a solvent inert to the reaction at a temperature of usually 0 to 100°C, preferably at 10 to 80°C, for 1 to 24 hours.

When a hydrazine salt is used as a reactant, the reaction can be accelerated in the presence of a base such as sodium acetate, sodium hydrogencarbonate or potassium carbonate.

The solvents for the reaction include aromatic hydrocarbons such as benzene, toluene and xylene; halogenated hydrocarbons such as dichloromethane, chloroform and 1,2-dichloroethane; ethers such as diethyl ether, tetrahydrofuran and dioxane; alcohols such as methanol, ethanol and propanol; and polar solvents such as N,N-dimethylformamide, N-methylpyrrolidone, dimethyl sulfoxide, acetic acid and water. They can be used either alone or in the form of a solvent mixture.

The dioxocarboxylic acid esters (II) used as a starting material can be obtained by the alcoholysis of dehydroacetic acid derivatives [Tetrahedron: Asymmetry, 1995, 6 (11), 2679, J. Chem. Soc., 1906, 89, 1186], carboxylation of acetylacetone (J. Org. Chem., 1966, 31, 1032, J. Chem. Soc. Perkin Trans., 1980, 2272), acylation of acetoacetic acid esters (Tetrahedron, 1995, 51 (47) 12859, Can. J. Chem., 1974, 52, 1343) or alcoholysis of Meltrum's acid derivatives (Synth. Commun., 1988, 18, 735).

The ethynyl derivatives (VI) used as a starting material in the above-described synthesis reaction can be synthesized according to the process described in J. Org. Chem., 50, 1763 (1985).

As described above, the insecticidal and acaricidal activity of the pyrazolyl derivatives of the general formula (I) have been unknown. The present invention provides such a novel use of them.

The pyrazolyl derivatives of the general formula (I) are highly effective in controlling hygienic vermin or insects harmful for agricultural and horticultural products even when they are used in a low concentration. The vermin and acarid which can be controlled are eggs, larvae and imagoes of, for example, Lepidoptera including tobacco cutworm (Spodoptera litura), diamond backmoth (Plutella xylostera), smaller tea tortix (Adoxophyes orana), grass leaf roller (Cnaphalocrocis medinalis) and rice stem borer (Chilo suppressalis); those of Hemiptera including leafhoppers such as brown rice planthopper (Nilaparvata lugens) and white backed rice planthopper (Sogatella furcifera), leafhoppers such as green rice leafhopper (Nephotettix cincticeps) and smaller green leafhopper (Chlorita flavescens), aphids such as green peach aphid (Myzus persicae) and cotton aphid (Aphis gossypii), whiteflies such as green house whitefly (Trialeurodes vaporariorum), and soldier bugs such as brownwinged green bug (Plautia stali); beetles such as striped flea beetle (phyllotreta striolata), cucurbit leaf beetle (Aulacophora femoralis) and adzuki bean weevil (Callosobruchus chinersis); Diptera such as housefly (Musca domestica) and house common mosquito (Culex pipiens fallens); Orthoptera such as American cockroach (Periplaneta americana); and acarids such as two spotted red spider (Tetranychus telarius), citrus red mite (Panonychus citri), citrus rust mite (Phyllocoprata oleivorus) and broad mite Polyphagotarsonemus latus).

The pyrazolyl derivatives of the general formula (I) are particularly excellent in the acaricidal activities, and they also exhibit excellent effect of controlling imagoes and eggs of mites.

When the compounds used according to the present invention are used as agricultural and horticultural insecticides and acaricides, they can be used as they are. However, it is preferred that they are used in the form of compositions containing a pesticide adjuvant generally used in the field of the pesticidal preparation. The form of the pesticidal preparation is not restricted. They are preferably used in the form of, for example, an emulsion, wettable powder, powder, flowable powder, granules, tablets, oil, spray or fumigant.

In the preparation of the insecticidal and acaricidal compositions, various agricultural adjuvants are used for the purposes of improving and stabilizing the effect, and also improving the dispersibility. The agricultural adjuvants which vary depending on the type of the preparation are usually carriers (diluents) such as liquid carriers and solid carriers; and surfactants.

The liquid carriers include, for example, water; aromatic hydrocarbons such as alkylbenzenes, e. g. toluene and xylene, alkylnaphthalenes, e. g. methylnaphthalene and dimethylnaphthalene, and chlorobenzene; alcohols such as methyl alcohol, ethyl alcohol, isopropyl alcohol, n-butyl alcohol and benzyl alcohol; halogenated hydrocarbons such as ethylene chloride, methylene chloride, chloroform and carbon tetrachloride; ketones such as acetone, methyl ethyl ketone, cyclohexanone and methyl isobutyl ketone; ethers such as ethyl ether, ethylene oxide and dioxane; esters such as ethyl acetate, amyl acetate, γ-butyrolactone and ethylene glycol acetate; nitriles such as acetonitrile and acrylonitrile; amides such as N,N-dimethylformamide, N,N-dimethylacetamide and N-methylpyrrolidone; sulfoxides such as dimethyl sulfoxide; alcohol ethers such as ethylene glycol monomethyl ether; aliphatic and alicyclic hydrocarbons such as n-hexane and cyclohexane; industrial gasolines such as petroleum ether and solvent naphtha; petroleum fractions such as paraffins, kerosene and gas oil; animal and vegetable oils; and fatty acids.

The solid carriers usable herein include mineral powders such as clay, kaolin, talc, diatomaceous earth, silica, calcium carbonate, montmorillonite, bentonite, feldspar and quartz; vegetable powders such as starch, crystalline cellulose and wheat flour; silicates, polysaccharides, alumina, highly dispersed silicic acid, waxes and gum arabic.

In the preparation of the emulsion, wettable powder, flowable powder, etc., a surfactant (or an emulsifier) is used for the purpose of improving the emulsification, dispersion, solubilization, wetting, foaming, lubrication or spreading. The surfactants include nonionic surfactants such as polyoxyethylene alkyl ethers, polyoxyethylene alkylallyl ethers, polyoxyethylene alkyl esters, polyoxyethylene castor oil ethers, polyoxyethylene sorbitan alkyl esters, sorbitan alkyl esters, carboxymethyl cellulose, polyvinyl alcohol and organic silicones, e. g. trisiloxane alkoxylates; anionic surfactants such as alkylbenzene sulfonates, alkyl sulfosuccinates, alkyl sulfates, polyoxyethylene alkyl sulfates, aryl sulfonates, sodium lignin sulfonate and sodium laurylsulfate; and cationic surfactants such as alkylammonium salts, e. g. cationic surfactant alkyldimethylbenzylammonium chlorides. The surfactants are used either alone or in the form of a mixture of two or more of them depending on the purpose.

The amount of the active ingredient of the present invention used for the preparation is suitably selected in the range of 0.1 to 99.5 % depending on the form of the preparation, application method and various other conditions. For example, the amount of the active ingredient is about 0.5 to 20 % by weight, preferably 1 to 10 % by weight, for the powder; about 1 to 90 % by weight, preferably 10 to 80 % by weight, for the wettable powder; and about 1 to 90 % by weight, preferably 10 to 40 % by weight, for the emulsion.

The preparations of the above described insecticide and acaricide are practically used as follows: For example, when the preparation is an emulsion, it is prepared by mixing the active ingredient, a solvent, a surfactant, etc. to obtain an undiluted emulsion, which is usually diluted with water to a predetermined concentration at the time of use. When the preparation is a wettable powder, it is prepared by mixing the active ingredient, a solid carrier, a surfactant, etc. to obtain an undiluted wettable powder, which is usually diluted with water to a predetermined concentration at the time of use. When the preparation is a powder, it is prepared by mixing the active ingredient, a solid carrier, etc. and the obtained powder is usually used as it is. When the preparation is in the form of granules, it is prepared by mixing the active ingredient, a solid carrier, a surfactant, etc. and then granulating the obtained mixture, and the obtained granules are usually used as they are. As a matter of course, processes for producing the various preparations are not limited to those described above. The processes can be suitably selected by those skilled in the art depending on the variety of the active ingredient and the purpose of the use.

The method for the application of the insecticide and acaricide is not particularly limited. They can be applied by any of the foliar application method, submerged application method, soil treatment method, seed treatment method, etc. For example, in the foliar application method, an aqueous solution of the insecticide and acaricide having a concentration in the range of 5 to 1,000 ppm, preferably 10 to 500 ppm, is applied in an amount of 50 to 500 liters, preferably 100 to 200 liters, for 10 ares. When granules containing 5 to 15 % of the active ingredient are used by the submerged application method, the amount thereof is 1 to 10 kg for 10 ares. In the soil treatment method, an aqueous solution having a concentration of 5 to 1000 ppm, preferably 10 to 500 ppm, is applied in an amount of 1 to 10 liters per m². In the seed treatment method, an aqueous solution having a concentration of 10 to 1000 ppm is applied in an amount of 10 to 100 liters per kg of the seeds.

According to the present invention, the insecticide and acaricide can be used in the form of a mixture with other active ingredients such as fungicides, insecticides and acaricides so far as they do not inhibit the insecticidal and acaricidal effects of the active ingredient of the agent.

When the pyrazolylacrylic acid derivative of the general formula (I) is used in combination with a suitable, known fungicide, insecticide or acaricide, the mutual complement in the control spectrum becomes possible to reduce the total number of times of the application and, as a result, to reduce the total amount of the active componds. This is a remarkable effect. Further, when the derivative used according to the present invention is employed in combination with a known fungicide, insecticide or acaricide having a different effect, the resistance towards each agent, the development of which is feared when they are used separately, can be inhibited or delayed.

The mixture can be prepared by mixing active ingredients, i.e. a pyrazolyl derivative of the general formula (I) and at least one of known fungicidal, insecticidal and acaricidal ingredients, with a suitable carrier and also an adjuvant such as an emulsifying agent, dispersing agent, stabilizer, suspending agent and penetrating agent to obtain a wettable powder, water-soluble powder, emulsion, liquid formulation, sol (flowable powder), oil, powder, granules or aerosol by an ordinary method. The carriers usable herein are either solid or liquid carriers usually used for pesticides. They are not limited to particular ones. In the preparation of the emulsion, wettable powder, sol, etc., a surfactant (or an emulsifying agent) is used for the purpose of the emulsification, dispersion, solubilization, wetting, foaming, lubrication, spreading or the like. The surfactant (or an emulsifying agent) is not particularly limited. In addition, various adjuvants and, if necessary, stabilizers such as antioxidants and ultraviolet absorbents, and coloring agents are usable.

As for the amount (%) of the active ingredient of the present invention in those preparations, it is in the range of 1 to 90 % (by weight; the same shall apply hereinafter) in the wettable powder, water-soluble powder, emulsion, liquid formulation and sol; 0.5 to 10 % in the oil, powder and granules; and 0.01 to 2 % in the aerosol.

The mixing ratio (wt. %) of the compound (1) used according to the present invention to the other fungicidal, insecticidal or acaricidal ingredient can be generally 1 0.01 to 99, preferably 1 / 0.1 to 20. These preparations are diluted to a suitable concentration and used for various purposes such as the foliar application, seed treatment, soil treatment, submerged application or direct application.

Examples of the pesticides usable in the form of a mixture with the compounds used according to the present invention are as follows:
triazole compounds such as (2RS,3SR)-1-[3-(2-chlorophenyl)-2,3-epoxy-2-(4-fluorophenyl)propyl]-1H-1,2,4-triazole, 1-(biphenyl-4-yloxy)-3,3-dimethyl-1-(1H-1,2,4-triazole-1-yl)butane-2-ol, 1-[(2RS,4RS:2RS,4SR)-4-bromo-2-(2,4-dichlorophenyl)tetrahydro furfuryl]-1H-1,2,4-triazole, bis(4-fluorophenyl)(methyl)(1H-1,2,4-triazole-1-ylmethyl)silane, (2RS,3RS;2RS,3SR)-2-(4-chlorophenyl)-3-cyclopropyl-1-(1H-1,2,4-triazole-1-yl)butane-2-ol, cis,trans-3-chloro-4-[4-mothyl-2-(1H-1,2,4-triazole-1-ylmethyl)-1,3-dioxolan-2-yl]phenyl 4-chlorophenyl ether, 4-(4-chlorophenyl)-2-phenyl-2-(1H-1,2,4-triazole-1-ylmethyl)butyronitrile, 3-(2,4-dichlorophenyl)-6-fluoro-2-(1H-1,2,4-triazole-1-yl)quinazoline-4(3H)-on, (RS)-2-(2,4-dichlorophenyl)-1-(1H-1,2,4-triazole-1-yl)hexane-2-ol, (1RS,5RS;1RS,5SR)-5-(4-chlorobenzyl)-2,2-dimethyl-1-(1H-1,2,4-triazole-1-ylmethyl)cyclopentanol, 2-p-chlorophenyl-2-(1H-1,2,4-triazole-1-ylmethyl)hexanenitrile, (±)-1-[2-(2,4-dichlorophenyl)-4-propyl-1,3-dioxirane-2-ylmethyl]-1H-1,2,4-triazole, (RS)-1-p-chlorophenyl-4,4-dimethyl-3-(1H-1,2,4-triazole-1-ylmethyl)pentane-3-ol, (RS)-2-(2,4-dichlorophenyl)-3-(1H-1,2,4-triazole-1-yl)propyl 1,1,2,2-tetrafluoroethyl ether, 1-(4-chlorophenoxy)-3,3-dimethyl-1-(1H-1,2,4-triazole-1-yl)butane-2-on and (1RS,2RS;1RS,2SR)-1-(4-chlorophenoxy)-3,3-dimethyl-1-(1H-1,2,4-triazole-1-yl)butane-2-ol; and azole compounds such as imidazole compounds, e. g. (E)-4-chloro- α,α,α-trifluoro-N-(1-imidazole-1-yl-2-propoxyethylidene)-o-toluidine, N-propyl-N-[2-(2,4,6-trichlorophenoxy)ethyl]imidazole-1-carboxamide and (±)-1-(β-allyloxy-2,4-dichlorophenylethyl)imidazole;
pyrimidine compounds such as (±)-2,4-dichloro-α-(pyrimidine-5-yl)benzhydryl alcohol and (±)-2-chloro-4'-fluoro-α-(pyrimidine-5-yl)benzhydryl alcohol;
morpholine compounds and morpholine derivatives such as (±)-cis-4-[3-(4-tert-butylphenyl)-2-methylpropyl]-2,6-dimethylmorpholine, 2,6-dimethyl-4-tridecylmorpholine and (RS)-1-[3-(4-tert-butylphenyl)-2-methylpropyl]piperidine;
benzimidazole compounds such as methyl 1-(butylcarbamoyl)benzimidazole-2-yl carbamate, dimethyl 4,4'-(o-phenylene) bis(3-thioallophanate), methyl benzimidazole-2-ylcarbamate and 2-(thiazole-4-yl)benzimidazole;
dicarboxyimide compounds such as N-(3,5-dichlorophenyl)-1,2-dimethylcyclopropane-1,2-dicarboxyimide, 3-(3,5-dichlorophenyl)-N-isopropyl-2,4-dioxoimidazolidine-1-carboxamide and (RS)-3-(3,5-dichlorophenyl)-5-methyl-5-vinyl-1,3-oxazolidine-2,4-dione; acylalanine compounds such as methyl N-(2-methoxyacetyl)-N-(2,6-xylyl)-DL-alaninate and 2-methoxy-N-(2-oxo-1,3-oxazolidine-3-yl)aceto-2',6'-xylidate;
organophosphorus compounds such as O-ethyl S,S-diphenyl phosphorodithioate and S-benzyl O,O-di-isopropylphosphorothioate;
phenylamide compounds such as 3'-isopropoxy-o-toluanilide, α,α, α-trifluoro-3'-isopropoxy-o-toluanilide and 5,6-dihydro-2-methyl-1,4-oxathi-ine-3-carboxanilide 4,4-dioxide; dithiocarbamate compounds such as zinc ion-coordinated manganese ethylene bisdithiocarbamate, manganese ethylene bisdithiocarbamate, zinc ion-coordinated ethylene bisdithiocarbamate and zinc ion-coordinated bisdimethyldithiocarbamate;
anilinopyrimidine compounds such as N-(4-methyl-6-propin-1-ylpyrimidine-2-yl)aniline, N-(4,6-dimethylpyrimidine-2-yl)aniline and 4-cyclopropyl-6-methyl-N-phenylpyrimidine-2-amine;
strobilurine derivatives such as methylmethoxyimino-α-(o-tolyloxy)-o-tolyl acetate and methyl (E)-{2-[6-(2-cyanophenoxy)pyrimidine-4-yloxy]phenyl}-3-methoxyacrylate; and antibiotics such as S,S-(6-methylquinoxaline-2,3-diyl) dithiocarbonate, 3-chloro-N-(3-chloro-5-trifluoromethyl-2-pyridyl)-α, α, α-trifluoro-2,6-dinitro-p-toluidine, tetrachloroisophthalonitrile, N-dichlorofluoromethyl thio-N,N'-dimethyl-N-phenylsulfamide, 1-(2-cyano-2-methoxyiminoacetyl)-3-ethylurea, aluminum tris(ethyl phosphonate), 2,3-dichloro-N-fluorophenyl maleimide, 5,10-dihydro-5,10-dioxonaphtho[2.3-b]-1,4-dithi-ine-2,3-dicarbonitrile, (E,Z)-4-[3-(4-chlorophenyl)-3-(3,4-dimethoxyphenyl)acryloyl]morpholine, 4-(2,2-difluoro-1,3-benzodioxol-4-yl)-lH-pyrrole-3-carbonitrile,
1,1'-iminodi(octamethylene)diguanidine, 4,5,6,7-tetrachlorophthalide, 3-allyloxy-1,2-benz[d]isothiazole 1,1-dioxide, 5-methyl-1,2,4-triazolo[3,4-b][1,3]benzothiazole, 1,2,5,6-tetrahydropyrrolo[3,2,1-ij]quinoline-4-on, di-isopropyl 1,3-dithiolane-2-ylidenemalonate and isopropyl 3,4-diethoxycarbanylate. However, the pesticides are not always limited to those listed above.

Examples of other insecticidal ingredients are as follows:
organophosphorus insecticides such as dimethyl-2,2,2-trichloro-1-hydroxyethyl phosphonate, O,O-diethyl O-2-isopropyl-6-methylpyrimidine-4-yl phosphorothioate, 2,2-dichlorovinyl dimethylphosphate and dimethyl 2,2,2-trichloro-1-hydroxyethylphosphonate;
carbamate insecticides such as 2-sec-butylphenylmethyl carbamate, 1-naphthylmethyl carbamate and 2-dimethylamino-5,6-dimethylpyrimidine-4-yldimethyl carbamate;
pyrethroid insecticides such as (RS)-α-cyano-3-phenoxybenzyl-N-(2-chloro-α,α,α-trifluoro-p-toluyl)-D-valinate, 2-(4-ethoxyphenyl)-2-methylpropyl 3-phenoxybenzyl ester and (RS)- α-cyano-3-phenoxybenzyl(1RS,3RS;1RS,3SR)-3-(2,2-dichlorovinyl)-2,2-dimethyl-cyclopropane carboxylate;
benzoylurea insecticides such as 1-(3,5-dichloro-2,4-difluorophenyl)-3-(2,6-difluorobenzoyl)urea, 1-(4-chlorophenyl)-3-(2,6-difluorobenzoyl)urea, 1-[3,5-dichloro-4-(3-chloro-5-trifuoromethyl)-2-pyridyloxy]phenyl]-3-(2,6-difluorobenzoyl)urea; as well as 4-bromo-2-(4-chlorophenyl)-1-ethoxymethyl-5-trifluoromethylpyrrole-3-carbonitrile, 1-(6-chloro-3-pyridylmethyl)-N-nitroimidazolidine-2-ylideneamine, N-tert-butyl-N'-(4-ethylbenzoyl)-3,5-dimethylbenzohydrazide, 1-tert-butyl-3-(2,6-di-isopropyl-4-phenoxyphenyl)thiourea, S,S'-(2-dimethylaminotrimethylene)bis(thiocarbamate) and various antibiotics. However, the insecticides are not always limited to those listed above.

As examples of the known acaricidal ingredients, there are various compounds such as N-(4-tert-butylbenzyl)-4-chloro-3-ethyl-1-methylpyrazole-5-carboxamide, 2-tert-butyl-5-(4-tert-butylbenzylthio)-4-chloropyridazine-3(2H)-on, tert-butyl(E)-α-(1,3-dimethyl-5-phenoxypyrazole-4-ylmethylene aminoxy)-p-toluate, 2,2,2-trichloro-1,1-bis(4-chlorophenyl)ethanol, 2-(4-tert-butylphenoxy)cyclohexylprop-2-ynyl sulfite, N-methylbis(2,4-xylyliminomethyl)amine, (4RS,5RS)-5-(4-chlorophenyl)-N-cyclohexyl-4-methyl-2-oxo-1,3-thiazolidine-3-carboxamide and 3,6-bis(2-chlorophenyl)-1,2,4,5-tetrazine. The acaricidal ingredients are not always limited to those listed above.

### <Examples>

The following Examples will further illustrate the present invention which by no means limit the scope of the present invention, within the gist of the invention.

### [Synthesis Example 1] Methyl α-{1,3-dimethyl-4-(4-trifluoromethylphenylethynyl)-5-pyrazole}-β-methoxyacrylate (synthesis of compound No. 3 in Table 1):

A solution of 70.0 g (0.376 mol) of isopropyl 3,5-dioxohexanoate in 100 ml of toluene was added dropwise to a solution of 19.1 g (0.414 mol) of methylhydrazine in 200 ml of toluene at an inner temperature of -10 to -5°C over 15 minutes. The obtained reaction mixture was stirred at room temperature for 3 hours and then divided into phases. The organic phase was washed with saturated aqueous sodium chloride solution and then dried over anhydrous sodium sulfate. The solvent was evaporated and the residue was distilled under reduced pressure to obtain 67.4 g of isopropyl 1,3-dimethylpyrazole-5-ylacetate.
b. p.: 95-96°C · 1.5 mmHg, Yield: 91.3 %
¹H-NMR δ (ppm): 1.25(6H,d), 2.22(3H,s), 3.58(2H,s), 3.75(3H,s), 5.01(1H,m), 5.95 (1H,s)

6.36 g (25 mmol) of iodine and 1.76 g (10 mmol) of iodic acid were added to a mixed solution of 9.81 g (50 mmol) of isopropyl 1,3-dimethylpyrazole-5-ylacetate in a mixture of 30 ml of acetic acid, 10 ml of water and 10 ml of 1,2-dichloroethane, and they were heated under reflux for 1.5 hours. After cooling, an aqueous sodium thiosulfate solution was added to the reaction mixture to decolor the mixture. After the concentration under reduced pressure, hexane was added to the obtained residue. Crystals thus obtained were collected by the filtration to obtain 9.72 g (30 mmol) of isopropyl 4-iodo-1,3-dimethylpyrazole-5-ylacetate in the form of a yellow powder. Yield: 60 %.

1.41 g (8.29 mmol) of p-trifluoromethylphenylacetylene was added to a solution in 10 ml of triethylamine of 2.22 g (6.89 mmol) of isopropyl 4-iodo-1,3-dimethylpyrazole-5-ylacetate, 90 mg (8.29 mmol) of Pd(PPh₃)₄ and 20 mg (0.16 mmol) of copper (I) iodide at 90°C over 10 minutes. The obtained mixture was heated under reflux at that temperature for 4 hours. After cooling to room temperature, the crystals thus formed were separated by the filtration. The filtrate was concentrated under reduced pressure. The residue was purified by the silica gel chromatography to obtain 2.23 g (6.12 mmol) of isopropyl α -{1-methyl-4-(4-trifluoromethylphenylethynyl)-5-pyrazole}-acetate in the form of a yellow oil. Yield: 89 %.

5 ml of 1,2-dimethoxyethane and 5 ml of methanol were added to 0.4 g (10 mmol) of 60 % NaH. The obtained solution was added to a solution of 1.71 g (4.69 mmol) of isopropyl α-{1-methyl-4-(4-trifluoromethylphenylethynyl)-5-pyrazole}-acetate in 5 ml of methyl formate. They were stirred at room temperature for 2 hours. 1.4 g (10 mmol) of potassium carbonate, 1.2 ml (10 mmol) of methyl iodide and 10 ml of DMF (N,N-dimethylformamide) were added thereto. They were stirred at room temperature overnight and then concentrated under reduced pressure. The residue was purified by the silica gel chromatography (hexane/ethyl acetate = 1/1) to obtain 0.54 g (1.43 mmol) of the title compound in the form of crystals. m. p.: 116 to 116.6°C. Yield: 30 %.

### [Synthesis Example 2] Methyl α -[1,3-dimethyl-4-{3,5-bis(trifluoromethyl)phenylethynyl}-5-pyrazole]-β-methoxyacrylate (Synthesis of compound No. 5 in Table 1):

A solution in 20 ml of triethylamine of 1.93 g (6.00 mmol) of isopropyl 4-iodo-1,3-dimethylpyrazole-5-ylacetate, 120 mg (0.104 mmol) of Pd(PPh₃)₄, 40 mg (0.210 mmol) of copper (I) iodide and 1.6 g (6.72 mmol) of 3,5-bis(trifluoromethyl)phenylacetylene was refluxed under heating at 90°C for 4 hours. After cooling to room temperature, the crystals thus formed were taken by the filtration. The filtrate was concentrated under reduced pressure, and the residue was purified by the silica gel chromatography to obtain 2.1 g (4.86 mmol) of isopropyl α-[1,3-dimethyl-4-{3,5-bis(trifluoromethyl)phenylethynyl}-5-pyrazole]-acetate in the form of yellow crystals. m. p. 140 to 143°C. Yield: 81 %.

5 ml of 1,2-dimethoxyethane and 5 ml of methanol were added to 0.4 g (10 mmol) of 60 % NaH. The obtained solution was added to a solution of 2.0 g (4.63 mmol) of isopropyl α-[1,3-dimethyl-4-{3,5-bis(trifluoromethyl)phenylethynyl}-5-pyrazole]-acetate in 5 ml of methyl formate. They were stirred at room temperature for 2 hours. 1.4 g (10 mmol) of potassium carbonate, 1.2 ml (10 mmol) of methyl iodide and 10 ml of DMF were added to the obtained mixture. They were stirred at room temperature overnight and then concentrated under reduced pressure. The residue was purified by the silica gel chromatography (hexane/ethyl acetate = 1/1) to obtain 1.07 g (2.4 mmol) of the title compound in the form of crystals. m. p.: 117.4 to 118°C. Yield: 52 %.

### [Synthesis Example 3] Methyl α-[1-ethyl-3-methyl-4-{3,5-bis(trifluoromethyl)phenylethynyl}-5-pyrazole]-β-methoxyacrylate (Synthesis of compound No. 21 in Table 1):

A solution of 1.5 g (25 mmol) of ethylhydrazine in 10 ml of toluene was added dropwise to a solution of 4.5 g (24.2 mmol) of isopropyl 3,5-dioxohexanoate in 20 ml of toluene at an inner temperature of -10 to -5°C over 5 minutes. The obtained reaction mixture was stirred at room temperature for 3 hours and then divided into phases. The organic phase was washed with saturated aqueous sodium chloride solution and then dried over anhydrous sodium sulfate. The solvent was evaporated and 4 g of isopropyl 1-ethyl-3-methylpyrazol-5-ylacetate was obtained (Kugelrohr). Yield: 79 %.

0.7 g (13 mmol) of sodium methylate was added to a solution of 4 g (19 mmol) of isopropyl 1-ethyl-3-methylpyrazole-5-ylacetate in 20 ml of methanol, and they were stirred at room temperature for 5 hours. 1 ml of acetic acid was added to the obtained mixture, and the obtained mixture was concentrated under reduced pressure. Ethyl acetate was added to the residue to divide it into phases. The organic phase was washed with saturated aqueous sodium chloride solution and then dried over anhydrous sodium sulfate. The solvent was evaporated, and the product was purified by the silica gel chromatography to obtain 2.9 g of methyl 1-ethyl-3-methylpyrazole-5-ylacetate. Yield: 83.6 %.

2.33 g (9.18 mmol) of iodine and 0.56 g (3.18 mmol) of iodic acid were added to a solution of 2.9 g (15.9 mmol) of methyl 1-ethyl-3-methylpyrazole-5-ylacetate in a mixture of 9 ml of acetic acid, 3 ml of water and 9 ml of 1,2-dichloroethane, and they were heated under reflux for 2 hours. After cooling, an aqueous sodium thiosulfate solution was added to the reaction mixture to decolor it. After the concentration under reduced pressure, ethyl acetate was added to the obtained residue to divide the mixture into phases. The organic phase was washed with saturated aqueous sodium chloride solution and then dried over anhydrous sodium sulfate. The solvent was evaporated, and hexane was added to the residue. Crystals thus obtained were collected by the filtration to obtain 2 g of methyl 1-ethyl-4-iodo-3-methylpyrazole-5-ylacetate in the form of a yellow powder. Yield: 37.4 %.

1.7 g (7.14 mmol) of 3,5-bistrifluoromethylphenylacetylene was added to a solution in 10 ml of triethylamine of 1 g (3 mmol) of methyl 1-ethyl-4-iodo-3-methylpyrazole-5-ylacetate, 0.1 g (0.45 mmol) of palladium (II) acetate, 0.1 g of copper iodide and 0.5 g (1.9 mmol) of triphenylphosphine at 90°C over 30 minutes. After heating under reflux at that temperature for 3 hours, the reaction mixture was cooled to room temperature. The crystals thus formed were collected by the filtration. The filtrate was concentrated under reduced pressure, and the residue was purified by the silica gel chromatography to obtain 0.6 g of methyl α-[1-ethyl-4-{3,5-bis(trifluoromethyl)phenylethynyl}-5-pyrazole]-acetate. Yield: 48.2 %.

0.1g (2.5 mmol) of 60 % NaH was added to a solution in 5 ml of DMF of 0.6 g (1.43 mmol) of methyl α-[1-ethyl-3-methyl-4-{3,5-bis(trifluoromethyl)phenylethynyl}-5-pyrazole]acetate and 5 ml of methyl formate under cooling with ice. 30 minutes after, the temperature was elevated to room temperature, and the obtained mixture was stirred for 5 hours. 0.3 g (2.17 mmol) of potassium carbonate and 0.32 g (2.54 mmol) of dimethyl sulfate were added to the mixture under cooling with ice. After stirring at room temperature for 3 hours, ethyl acetate and water were added thereto to divide the mixture into phases. The organic phase was washed with water and then with saturated aqueous sodium chloride solution, and dried over anhydrous sodium sulfate. The solvent was evaporated, and the residue was purified by the silica gel chromatography (hexane/ethyl acetate = 2/1) to obtain 0.35 g of the title compound (m. p.: 119.8 to 120.5°C, yield: 53 %) in the E form of crystals and 0.06 g (yield: 9 %) of the title compound in the Z form of a viscous liquid.

### [Synthesis Example 4] Methyl α-[1-methyl-3-trifluoromethyl-4-{3,5-bis(trifluoromethyl)phenylethynyl}-5-pyrazole]-β-methoxyacrylate (Synthesis of compound No. 20 in Table 1):

Palladium (II) acetate (0.7 g), copper (I) iodide (0.3 g), triphenylphosphine (3.3 g) and 1.5 g of active carbon were added to a solution of 8.6 g (24.7 mmol) of methyl 1-methyl-4-iodo-3-trifluoromethylpyrazole-5-ylacetate in a mixture of 10 ml of triethylamine and 20 ml of DMF, and they were stirred in nitrogen atmosphere for 30 minutes.

Then, 17 g (71.4 mmol) of 3,5-bistrifluoromethylphenylacetylene was added to the obtained mixture at 85 to 90°C over 40 minutes. They were heated under reflux at 90°C for 2 hours and then cooled to room temperature. The insoluble matter was removed by the filtration through Celite. Ethyl acetate and water were added to the filtrate to divide the obtained mixture into phases. The organic phase was washed with saturated aqueous sodium chloride solution and then dried over anhydrous sodium sulfate. After the concentration under reduced pressure, the residue was purified by the silica gel chromatography to obtain 9 g of methyl α -{1-methyl-3-trifluoromethyl-4-(3,5-bistrifluoromethylphenylethynyl)-5-pyrazole}-acetate (79 %).

1.17 g (29-25 mmol) of 60 % NaH was added to a solution in 45 ml of DMF of 9 g (19.62 mmol) of methyl α-{1-methyl-3-trifluoromethyl-4-(3,5-bistrifluoromethylphenylethynyl)-5-pyrazole}-acetate and 45 ml of methyl formate under cooling with ice. After stirring at 10°C for 1 hour and then at 25°C for 4 hours; 5.4 g (38.7 mmol) of potassium carbonate and 8.37 g (59.4 mmol) of methyl iodide were added to the reaction mixture, and they were stirred at 30°C for 5 hours. Ethyl acetate and water were added to the reaction mixture to divide the mixture into phases. The organic phase was washed with water and saturated aqueous sodium chloride solution and then dried over anhydrous sodium sulfate. The solvent was evaporated, and the residue was purified by the silica gel chromatography, and then the obtained crystals were recrystallized from hexane to obtain 7.65 g (78 %) of the title compound.

### [Referential Example 1] Synthesis of isopropyl 3,5-dioxohexanoate:

97 ml (0.69 mol) of triethylamine was added to a mixture of 100 g (0.69 mol) of Meldrum's acid and 350 ml of dichloromethane. Then, 76 ml (0.83 mol) of diketene was added dropwise thereto under cooling with ice over 15 minutes. After the completion of the addition, they were stirred at room temperature for 2 hours. Dilute hydrochloric acid was added to the reaction solution to divide it into phases. The organic phase was washed with water and saturated aqueous sodium chloride solution and then dried over anhydrous sodium sulfate. The solvent was evaporated, and the obtained crystals were washed with hexane/ethyl acetate (6/1)) and then dried under reduced pressure to obtain 137.4 g of acylated Meldrum's acid. m. p. 53-60°C. Yield: 87.3 %.

120 g (0.526 mol) of the acylation product obtained as described above and a solution of 94.7 g (1.58 mol) of 2-propanol in 1,000 ml of toluene were heated under reflux for 5 hours. The solvent was evaporated, and the residue was distilled under reduced pressure to obtain 70.1 g of isopropyl 3,5-dioxohexanoate. b. p.: 90-91°C 2.0 mmHg. Yield: 71.6 %.

### [Referential Example 2] Synthesis of 3,5-bistrifluoromethylphenylacetylene:

50.0 g (171 mmol) of 3,5-bistrifluoromethylbromobenzene was dissolved in 100 ml of triethylamine. 0.25 g (1.12 mmol) of palladium (II) acetate, 0.25 g (1.32 mmol) of copper (I) iodide and 1.0 g (3.83 mmol) of triphenylphosphine were added to the obtained solution at room temperature, and they were stirred at 30°C for 30 minutes. Then, 14.65 g (174 mmol) of 3-methyl-1-butyne-3-ol was added dropwise to the reaction mixture at a temperature in the range of 33 to 45°C over 2 hours. The reaction mixture was stirred at a temperature in the range of 35 to 40°C over 5.5 hours and then cooled. The salt thus precipitated was removed by the filtration. After thoroughly washing with ethyl acetate, the filtrate was washed with semi-saturated aqueous sodium chloride solution (700 ml + 500 ml) and saturated aqueous sodium chloride solution (100 ml x 2). The organic layer was dried over magnesium sulfate and then the solvent was evaporated under reduced pressure to obtain 49.9 g of crude 3-methyl-1-(3,5-bistrifluoromethylphenyl)-1-butyne-3-ol. Yield of the crude product: 98.5 %. Melting point: 74.5 to 74.8°C.

50 ml of liquid paraffin and 4.32 g (0.077 mol) of potassium hydroxide were added to crude 3-methyl-1-(3,5-bistrifluoromethylphenyl)-1-butyne-3-ol obtained as described above. The temperature of the oil bath was elevated to 85°C. Immediately thereafter, the pressure in the system was reduced to about 10mmHg with a vacuum pump. The fraction obtained through Vigreaux column was trapped in a flask cooled with solid carbon dioxide/acetone. The amount of the distillate was 40.9 g. The fraction was dissolved in 120 ml of diethyl ether and then washed with saturated aqueous sodium chloride solution (200 ml x 2). By this process, acetone in the distillate could be substantially completely removed. The organic layer was dried over magnesium sulfate and then evaporated by keeping the temperature of the water bath at 20°C to obtain 30.8 g of the title compound in the form of a colorless oily substance. Yield: 75.6 % (2 steps). nD = 1.4320.

Compounds produced by the processes illustrated in the above Examples or processes similar to them are shown in Table 1. Compounds produced by processes described in EP 433 899 and J. P. KOKAI Nos. Hei 5-201980, Hei 7-224041 and Hei 7-258219 or processes similar to them are shown in Tables 2, 3 and 4, which by no means limit the compounds used according to the present invention.

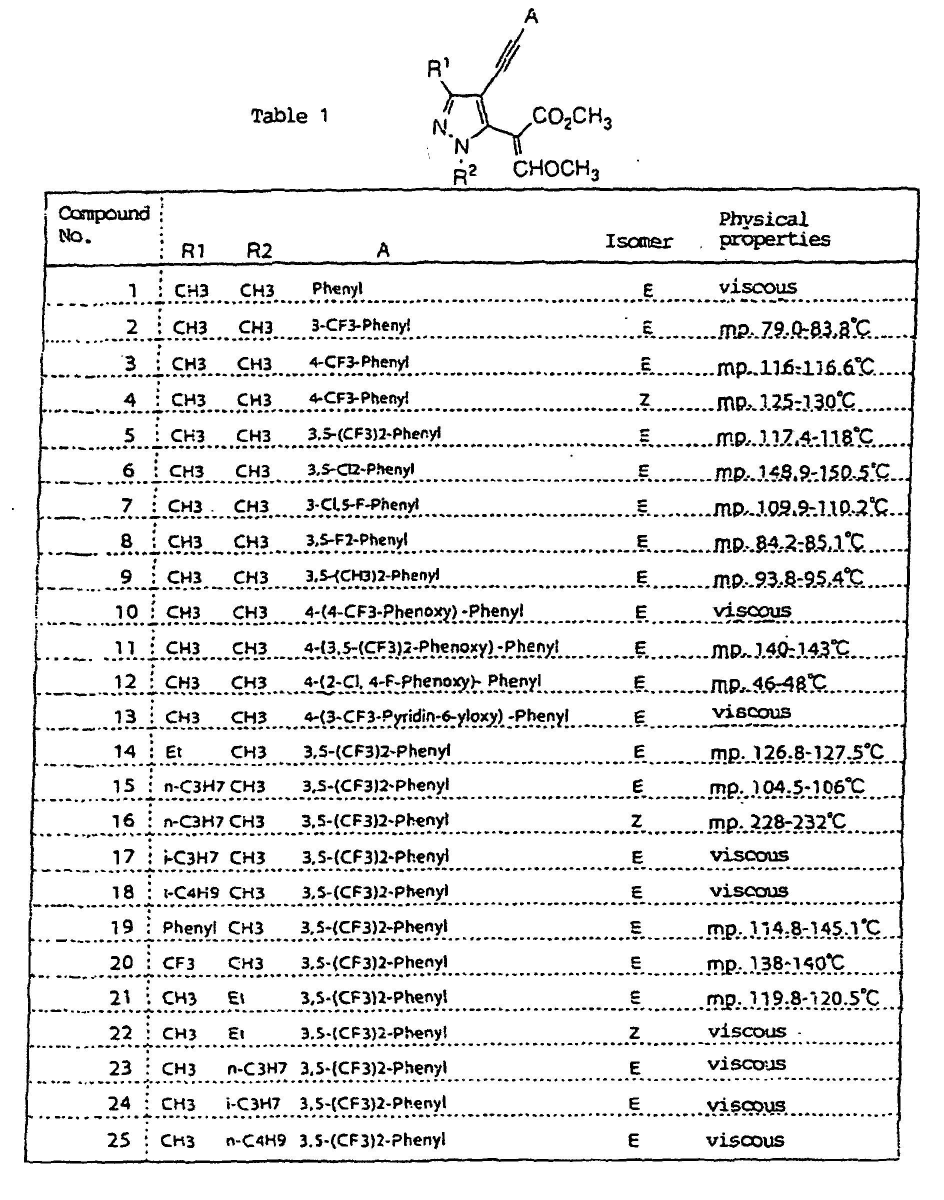

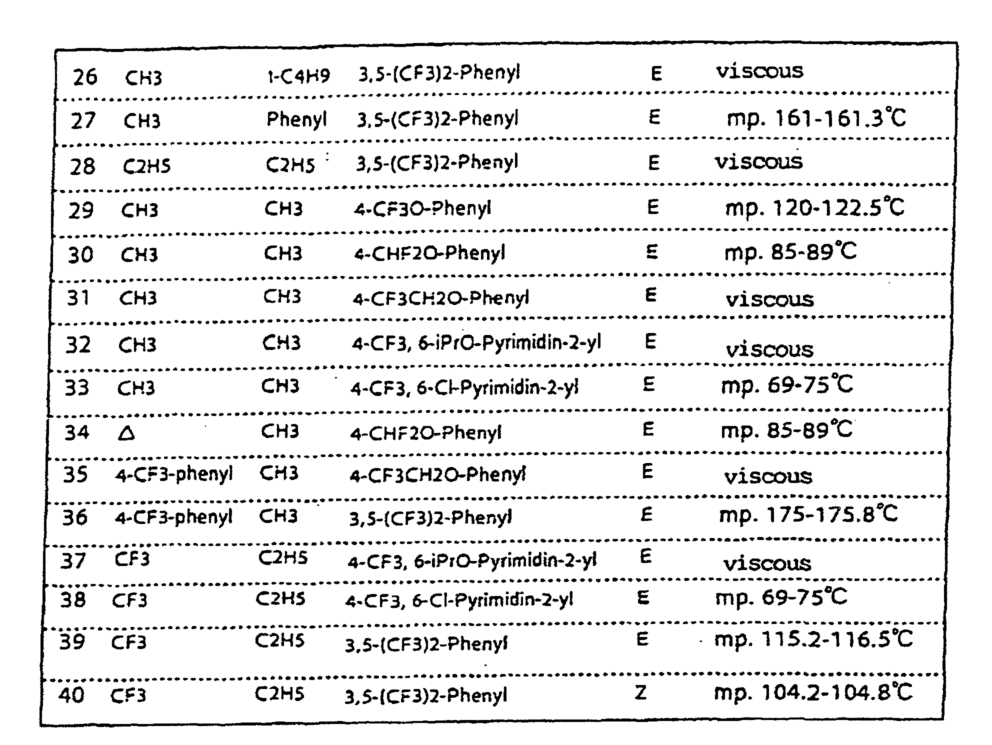

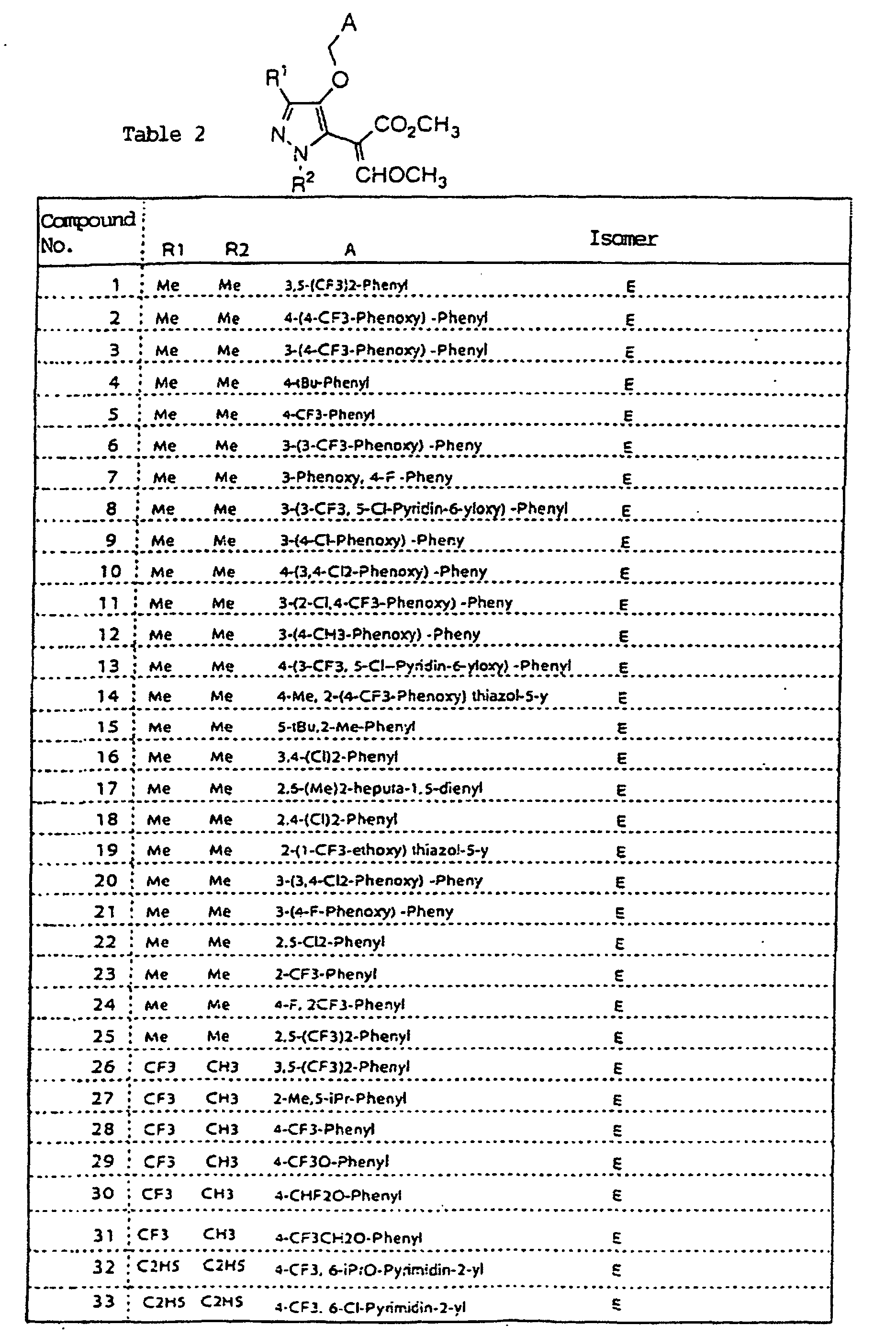

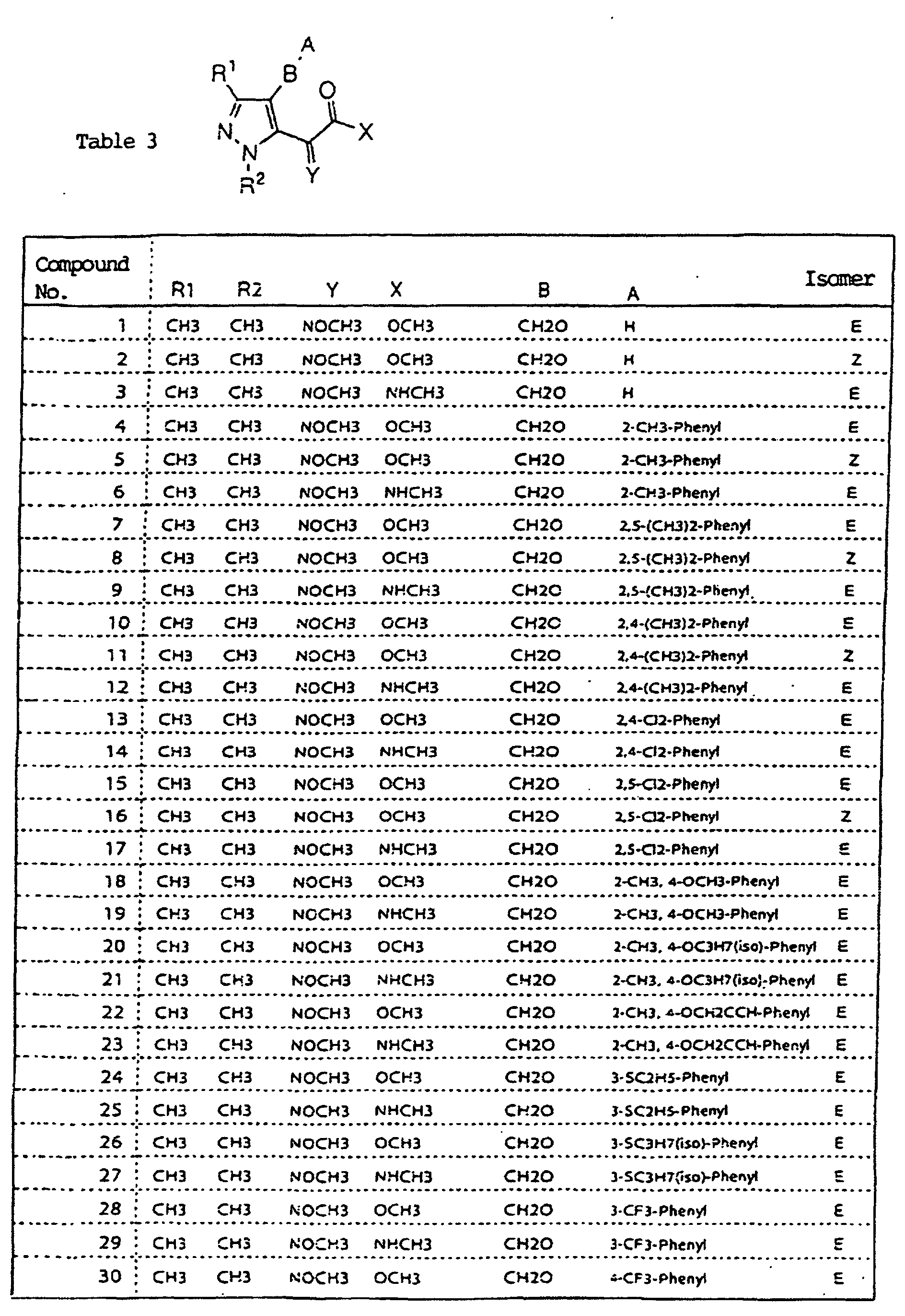

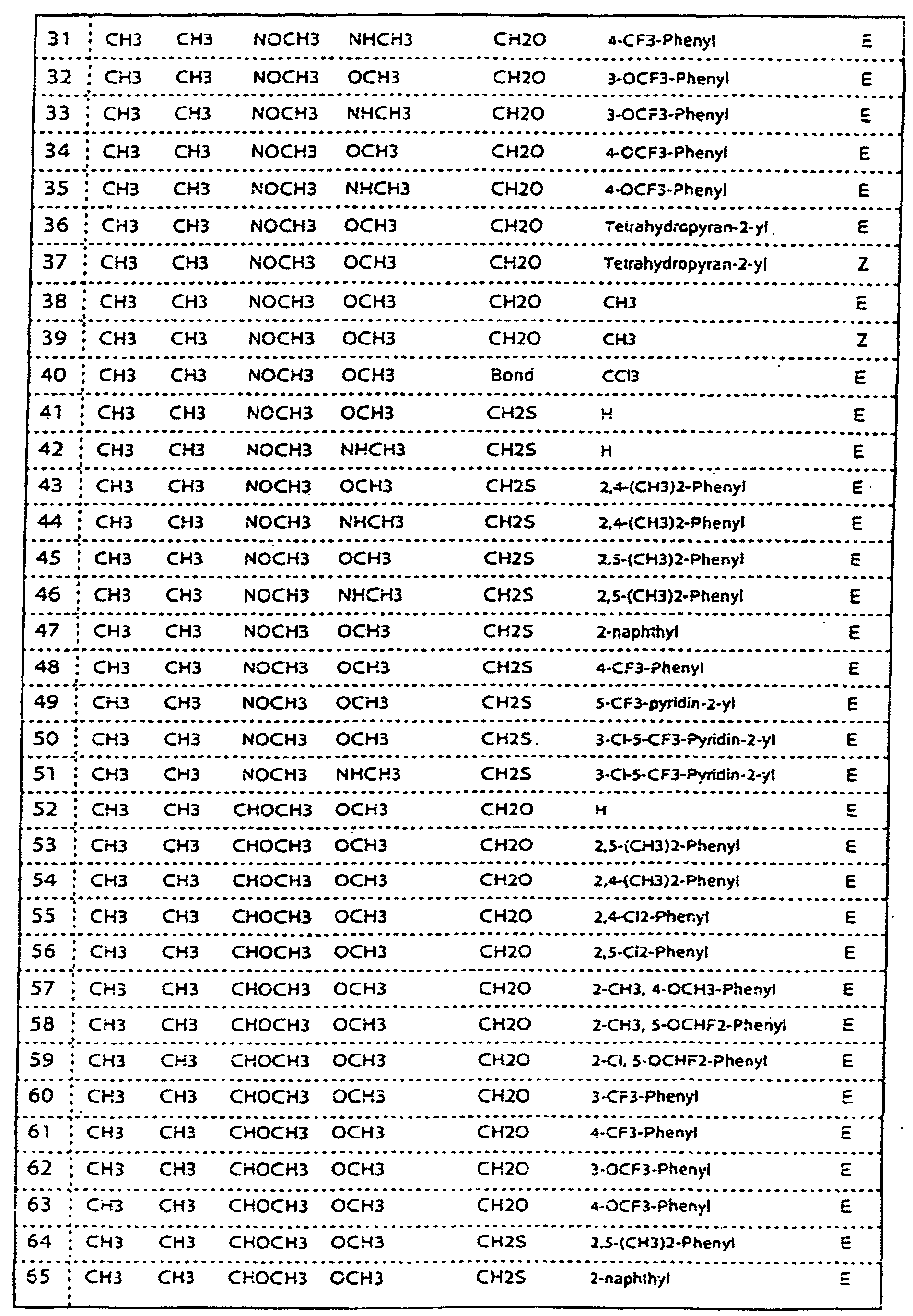

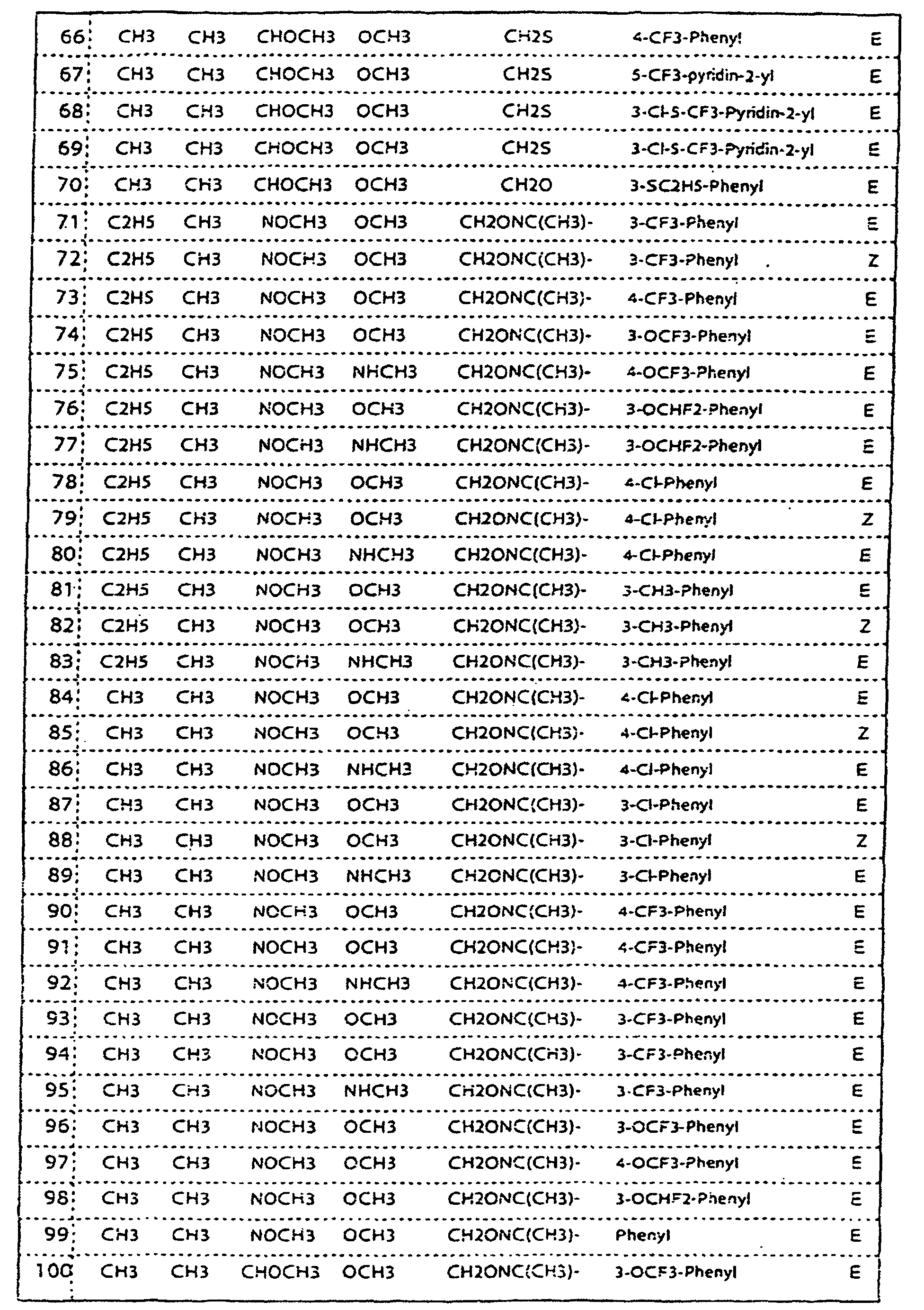

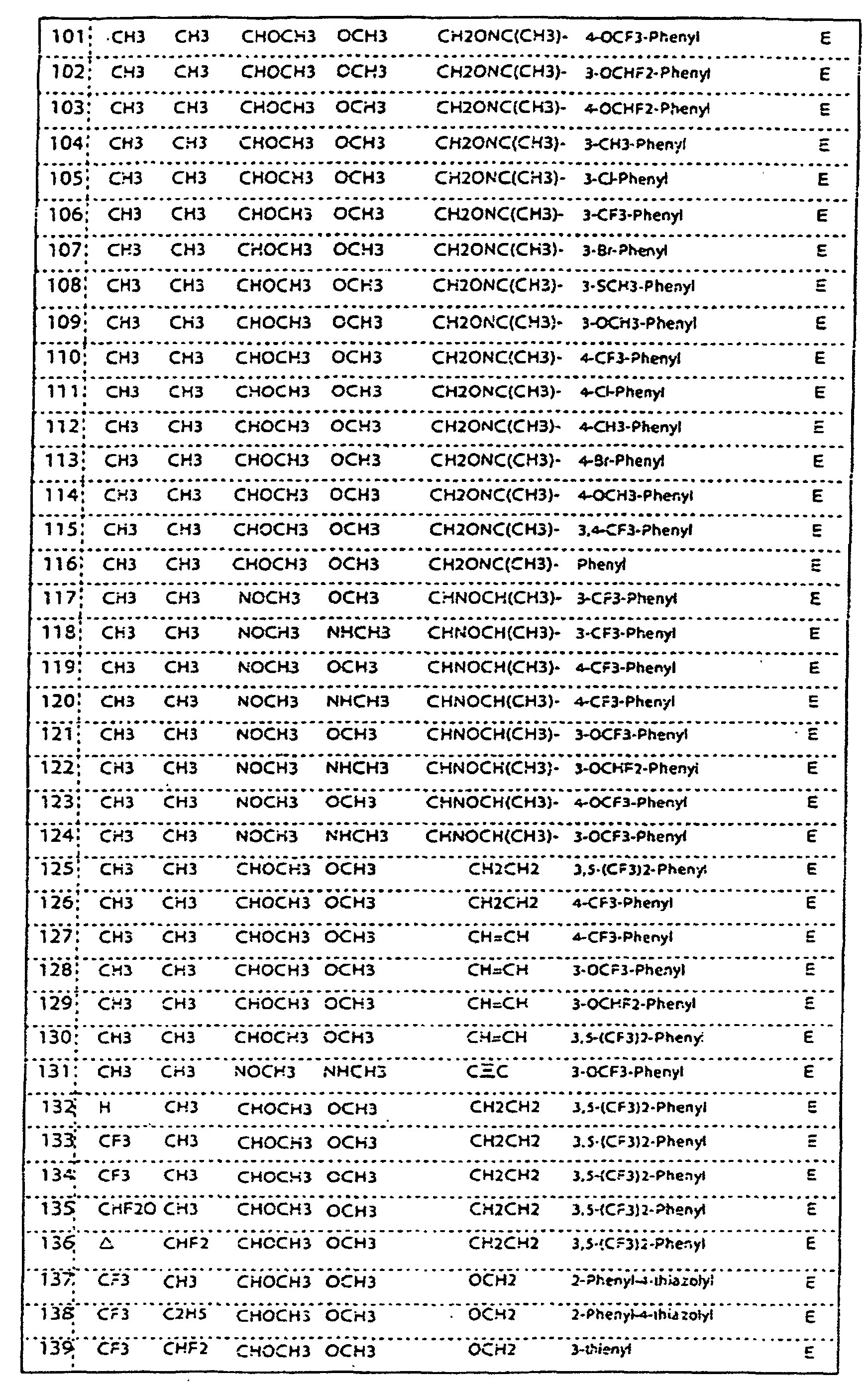

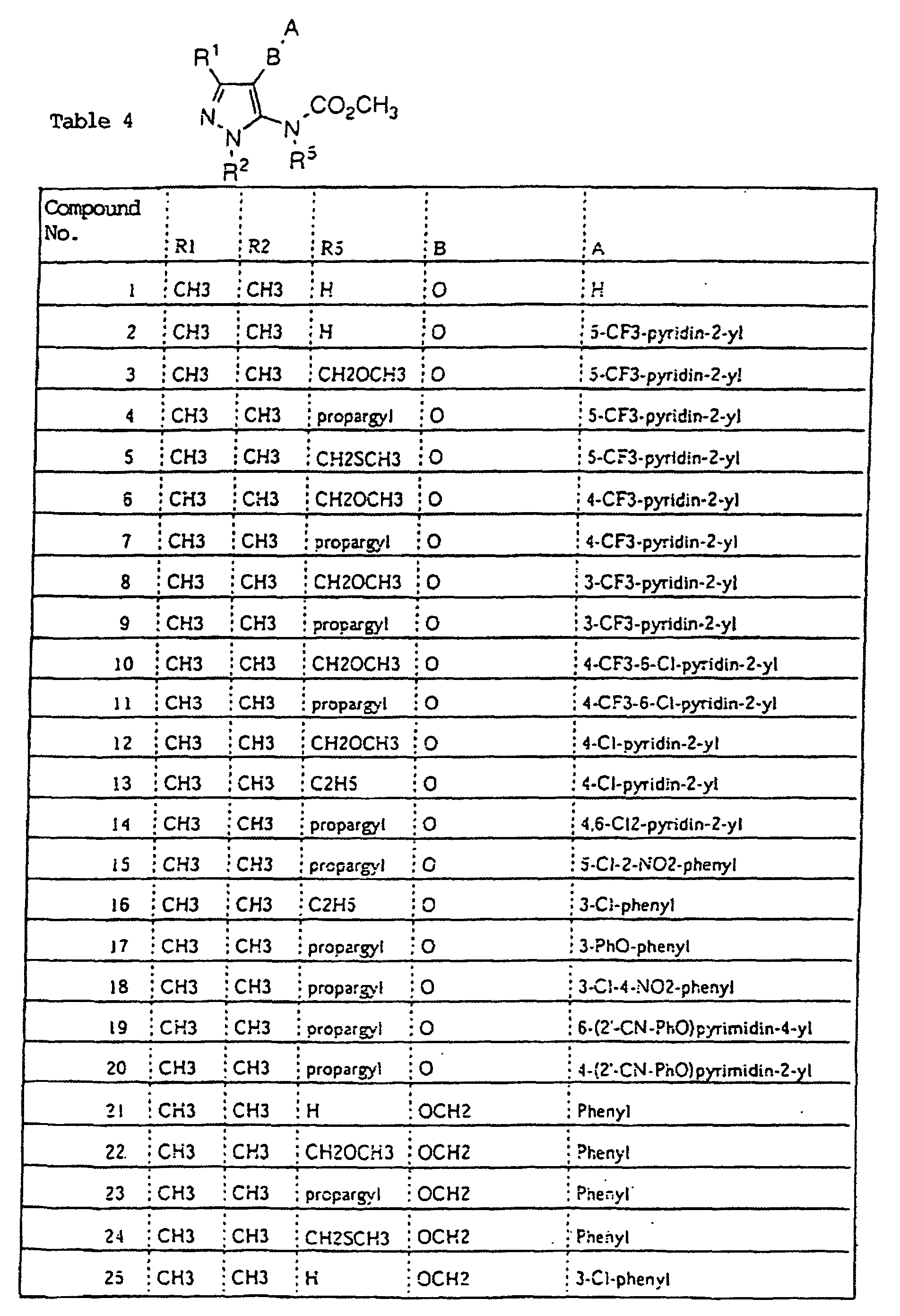

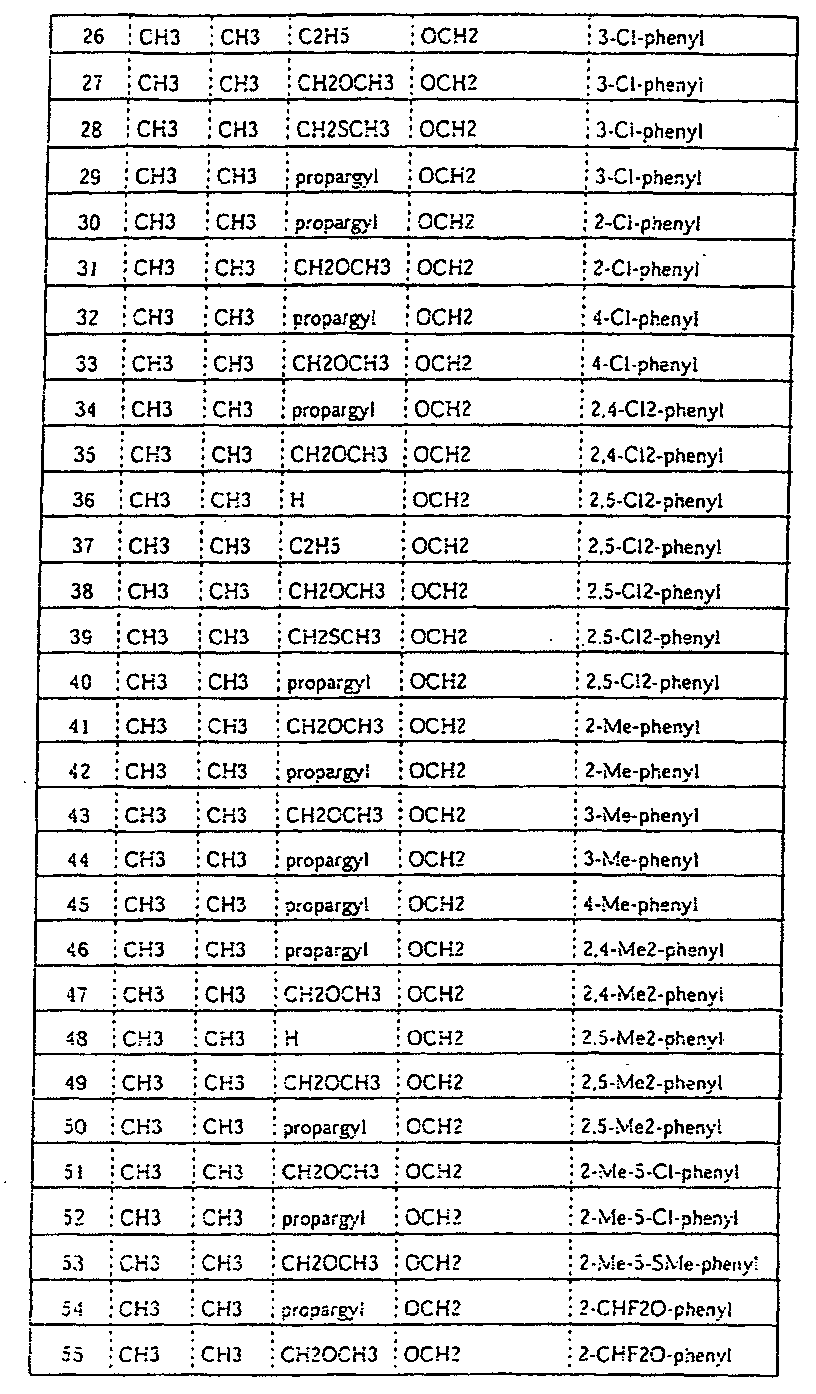

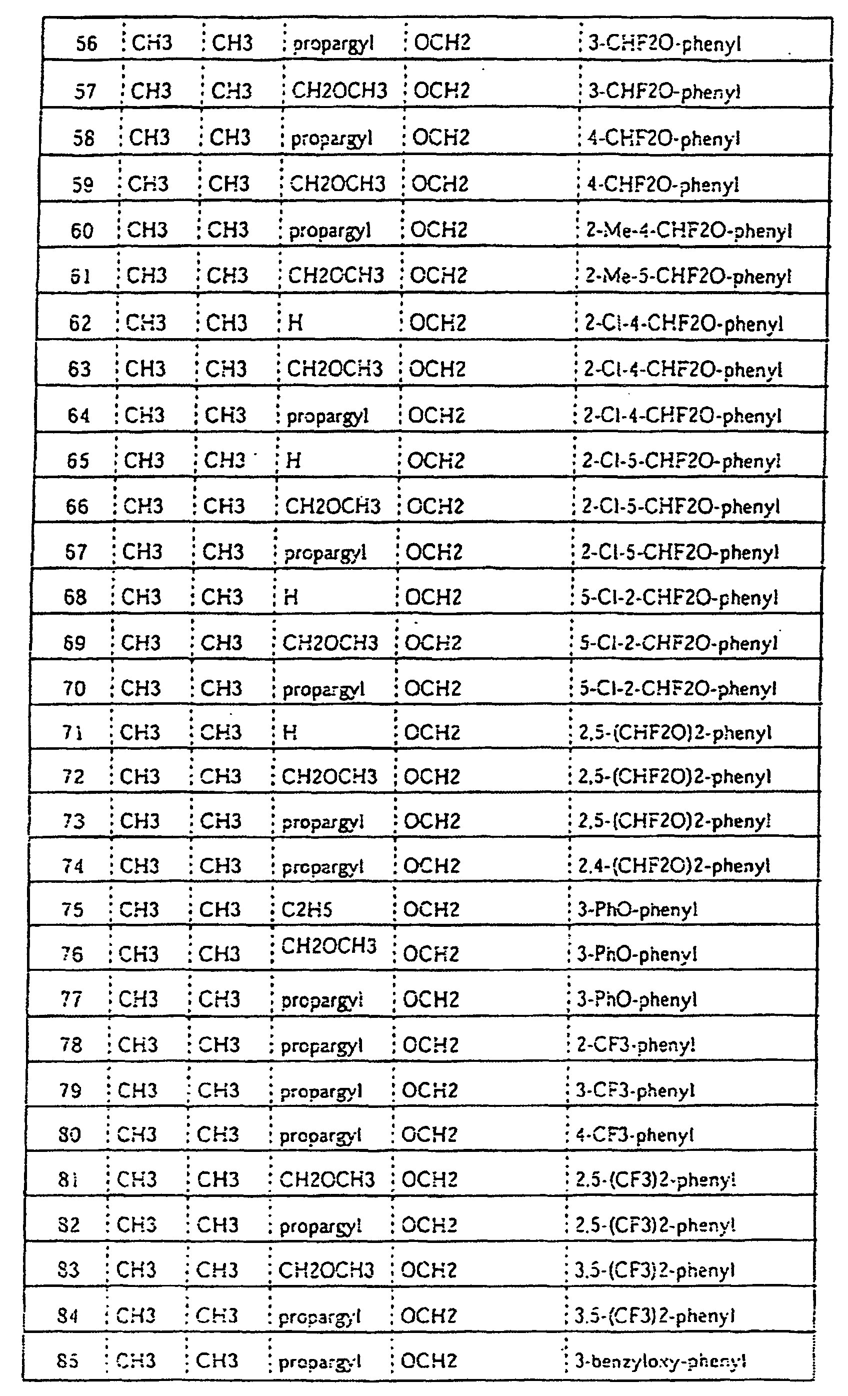

### [Preparation Example 1] Wettable powder:

20 parts by weight of the present compound was homogeneously mixed with 20 parts by weight of Carplex #80 (trade name of white carbon; Shionogi & Co., Ltd.), 52 parts by weight of ST kaolin clay (trade name of kaolinite; Tsuchiya Kaolin Co.), 5 parts of Sorpol 9047K (trade name of an anionic surfactant; Toho Chemical Industry Co., Ltd.) and 3 parts by weight of Runox P65L (trade name of an anionic surfactant; Toho Chemical Industry Co., Ltd.), and the mixture was pulverized to obtain a wettable powder containing 20 % by weight of the active ingredient.

### [Preparation Example 2] Powder:

2 parts by weight of the present compound was homogeneously mixed with 93 parts by weight of clay (a product of Nippon Talc) and 5 parts by weight of Carplex #80 (trade name of white carbon; Shionogi & Co., Ltd.), and the mixture was pulverized to obtain a powder containing 2 % by weight of the active ingredient.

### [Preparation Example 3] Emulsion:

20 parts by weight of the present compound was added to a solvent mixture comprising 35 parts by weight of xylene and 30 parts by weight of dimethylformamide to obtain a solution. 15 parts by weight of Sorpol 3005X (trade name of a mixture of a nonionic surfactant and an anionic surfactant; Toho Chemical Industry Co., Ltd.) was added to the solution to obtain an emulsion containing 20 % by weight of the active ingredient.

### [Preparation Example 4] Flowable powder:

30 parts by weight of a compound used according to the present invention was pulverized in wet method with 5 parts of Sorpol 9047K (see above), 3 parts by weight of Sorbon T-20 (trade name of a nonionic surfactant; Toho Chemical Industry Co., Ltd.), 8 parts by weight of ethylene glycol and 44 parts by weight of water with Dyno-mill (a product of Shinmaru Enterprises Co.). 10 parts by weight of 1 wt. % aqueous solution of xantham gum (natural high-molecular substance) was added to the obtained slurry mixture. They were thoroughly mixed and pulverized to obtain a flowable powder containing 20 % by weight of the active ingredient.

The following Test Examples will illustrate the usefulness of the present compounds as insecticides and acaricides.

### [Test Example 1] Insecticidal effect on larvae of diamond backmoth (Plutella xylostera):

The present insecticide prepared according to the Preparation Example was diluted with water. A piece (diameter: 6 cm) of a cabbage leaf was immersed in the diluted insecticide for 1 minute. After airdrying, the piece was placed in a plastic cup (inner diameter: 7 cm). 5 third-instar larvae of diamond backmoth (Plutella xylostera) were put in the cup (1 concentration, repetition: twice). The cup was kept in a constant temperature room at 25°C. On the fourth day, the survival rate and degree of writhing of the larvae were examined. The writhing larvae were each counted to be 1/2. The insecticidal rate (%) was determined to obtain the results shown in Table 5.

In the following Tables, the numbers of the compounds are the same as those in Tables 1, 2, 3 and 4.

**Table 5**

| Compound No. | Concentration(ppm) | Killing rate |
|---|---|---|
| Table 1-2 | 500 | 60 |
| Table 1-3 | 500 | 80 |
| Table 1-5 | 500 | 100 |
| Table 1-7 | 500 | 80 |
| Table 1-8 | 500 | 100 |
| Table 1-10 | 500 | 100 |
| Table 1-11 | 500 | 100 |
| Table 1-12 | 500 | 100 |
| Table 1-13 | 500 | 100 |
| Table 1-14 | 500 | 74 |
| Table 1-17 | 500 | 89 |
| Table 1-21 | 500 | 100 |
| Table 1-22 | 500 | 90 |
| Table 1-29 | 500 | 85 |
| Table 2-1 | 500 | 87 |
| Table 2-3 | 500 | 100 |
| Table 2-4 | 500 | 70 |
| Table 2-5 | 500 | 90 |
| Table 3-73 | 500 | 100 |
| Table 3-76 | 500 | 100 |
| Table 3-101 | 500 | 100 |

### [Test Example 2] Acaricidal effect on larvae of two-spotted red spiders (Tetranychus telarius):

A stem of green bean seedling having only one primary left was placed in a test tube (capacity: 50 ml) containing water. 15 female imagoes of two-spotted red spiders were put on each leaf. One day after, the leaf having the two-spotted red spiders was immersed (about 5 seconds) in the present insecticide diluted with water, which had been prepared according to the Preparation Example, (concentration: 500 ppm, repetition: twice). They were kept in a constant-temperature room at 25°C. On the fifth day after the treatment, the female imagoes on the leaf of the green bean seedling were observed. The imago-killing rate (%) was determined on the basis of the results of the observation to obtain the results shown in Table 6.

### [Test Example 3] Acaricidal effect on eggs of two-spotted red spiders (Tetranychus telarius):

5 female imagoes of two-spotted red spiders were released on a green been leaf disc (diameter: 3 cm). Then, the imagoes were left to lay eggs on the leaf disc for 20 hours and then removed therefrom. The present acaricide which had been prepared according to Preparation Example 1 was diluted with water to a predetermined concentration. 3.5 ml of the preparation thus obtained was sprayed with a rotary spraying column (Mizuho Rika) over the disc (concentration: 500 ppm, repetition: twice). 8 days after the treatment, the number of unhatched eggs was counted to determine the ovicidal rate (%). The results are shown in Table 6.

**Table 6**

| Compound No. | Acaricidal rate (%) | Ovicidal rate (%) |
|---|---|---|
| Table 1-2 | 100 | 100 |
| Table 1-3 | 100 | 100 |
| Table 1-5 | 100 | 100 |
| Table 1-6 | 100 | 100 |
| Table 1-7 | 100 | 100 |
| Table 1-8 | 100 | 100 |
| Table 1-9 | 96 | 80 |
| Table 1-10 | 100 | 100 |
| Table 1-11 | 100 | 100 |
| Table 1-12 | 100 | 100 |
| Table 1-13 | 100 | 100 |
| Table 1-14 | 100 | 100 |
| Table 1-15 | 100 | 100 |
| Table 1-16 | 100 | 100 |
| Table 1-17 | 100 | 100 |
| Table 1-18 | 100 | 100 |
| Table 1-19 | 100 | 100 |
| Table 1-20 | 100 | 100 |
| Table 1-21 | 100 | 100 |
| Table 1-22 | 100 | 100 |
| Table 1-28 | 100 | 100 |
| Table 1-29 | 100 | 100 |
| Table 1-30 | 100 | 100 |
| Table 1-37 | 100 | 100 |
| Table 1-39 | 100 | 100 |
| Table 1-40 | 100 | 100 |
| Table 2-1 | 100 | 100 |
| Table 2-2 | 100 | 100 |
| Table 2-3 | 100 | 100 |
| Table 2-4 | 100 | 100 |
| Table 2-5 | 100 | 100 |
| Table 2-6 | 100 | 100 |
| Table 2-7 | 100 | 100 |
| Table 2-8 | 100 | 100 |
| Table 2-9 | 100 | 100 |
| Table 2-10 | 100 | 100 |
| Table 2-11 | 100 | 100 |
| Table 2-12 | 100 | 100 |
| Table 2-13 | 100 | 100 |
| Table 2-14 | 100 | 100 |
| Table 2-15 | 100 | 100 |
| Table 2-16 | 96 | 80 |
| Table 2-17 | 100 | 100 |
| Table 2-18 | 100 | 100 |
| Table 2-19 | 100 | 100 |
| Table 2-20 | 100 | 100 |
| Table 2-21 | 100 | 100 |
| Table 2-22 | 100 | 100 |
| Table 2-23 | 100 | 100 |
| Table 2-24 | 100 | 100 |
| Table 2-25 | 100 | 100 |
| Table 2-26 | 100 | 100 |
| Table 2-29 | 100 | 100 |
| Table 2-30 | 100 | 100 |
| Table 2-32 | 100 | 100 |
| Table 2-33 | 100 | 100 |
| Table 3-61 | 100 | 100 |
| Table 3-63 | 100 | 100 |
| Table 3-66 | 100 | 100 |
| Table 3-75 | 100 | 100 |
| Table 3-90 | 100 | 100 |
| Table 3-92 | 100 | 100 |
| Table 3-101 | 100 | 100 |
| Table 3-102 | 100 | 100 |
| Table 3-115 | 100 | 100 |
| Table 4-23 | 100 | 100 |
| Table 4-40 | 100 | 100 |
| Table 4-85 | 100 | 100 |

### [Test Example 4] Acaricidal effect of a low concentration of acaricide on larvae of two-spotted red spiders (Tetranychus telarius):

The same tests as those of Test Example 2 were repeated except that the concentration of the acaricide was changed to 12.5 ppm. The following compounds exhibited 100 % acaricidal effect: Table 1-3, Table 1-5, Table 1-6, Table 1-7, Table 1-8, Table 1-10, Table 1-11, Table 1-12, Table 1-14, Table 1-15, Table 1-16, Table 1-17, Table 1-18, Table 1-19, Table 1-20, Table 1-21, Table 1-22, Table 1-28, Table 1-29, Table 1-30, Table 1-36, Table 1-37, Table 1-39, Table 1-40, Table 2-1 and Table 2-26.

### <Industrial Applicability>

The pyrazolyl derivatives used according to the present invention as insecticide and acaricide have an extremely excellent effect of controlling harmful insects and acarids in the agricultural and horticultural fields. They are useful as insecticides and acaricides in the agricultural and horticultural fields.

## Claims

1. Use of a pyrazolyl derivative of the following general formula (I): 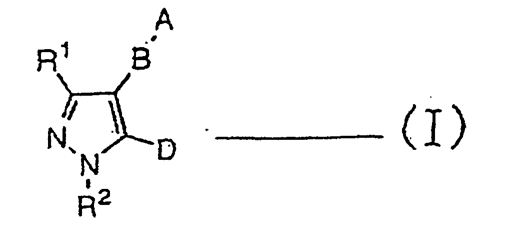 wherein
A represents a hydrogen atom; an alkyl group which may be substituted; an alkenyl group which may be substituted; an alkynyl group which may be substituted; a tri-substituted silyl group substituted with an alkyl group and/or an aryl group; an aryl group which may be substituted; or a heterocyclic group which may be substituted;
B represents a single bond; a group of the formula: -(G¹)ₙ-G²-(G¹)ₘ- wherein G¹ represents an oxygen atom, a sulfur atom, a sulfinyl group or a sulfonyl group, G² represents an alkylene group, an alkenylene group or an alkynylene group, and n and m are independent from each other and represent 0 or 1; a carbonyl group; a group of the formula: -CH₂-O-N=C(R³)- wherein R³ represents a hydrogen atom, an alkyl group or a haloalkyl group; or a group of the formula: -CH=N-O-(CR³R⁴)ₙ- wherein R³ and R⁴ each represents a hydrogen atom, an alkyl group or a haloalkyl group; and n is 0 or 1,
R¹ represents a hydrogen atom; a halogen atom, an alkyl group which may be substituted; an alkenyl group which may be substituted; an alkynyl group which may be substituted; an alkoxy group which may be substituted; or an aryl group which may be substituted,
R² represents a hydrogen atom; an alkyl group; a haloalkyl group; or an aryl group which may be substituted, and
D represents a group of the formula: -C(=Y)COX wherein X represents a hydroxyl group, an alkoxy group or an alkylamino group, Y represents a group of the formula: CH-(G³)ₙ-G⁴ wherein G³ represents an oxygen atom or a sulfur atom, G⁴ represents an alkyl group or a haloalkyl group, and n represents 0 or 1, a group of the formula: N-O-G⁴ wherein G⁴ represents an alkyl group or a haloalkyl group; or a group of the formula: -N(R⁵)CO₂G⁵ wherein R⁵ represents an alkyl group, an alkenyl group, an alkynyl group, an alkylthioalkyl group or an alkoxyalkyl group, and G⁵ represents an alkyl group; as an insecticide or acaricide.

2. Use according to claim 1, wherein:
B represents a group of the formula: -OCH₂-, -CH₂-O-, -C=C-; -CH=CH-; - CH₂CH₂-; a group of the formula: -CH₂ON=C(R³)- wherein R³ represents a hydrogen atom, an alkyl group or a haloalkyl group; or a group of the formula: -CH=NO-(CR³R⁴)ₙ- wherein R³ and R⁴ each represents a hydrogen atom, an alkyl group or a haloalkyl group; and n is 0 or 1,
R¹ represents a hydrogen atom, a halogen atom, an alkyl group, a haloalkyl group, an alkoxy group, a haloalkoxy group, or an aryl group which may be substituted,
R² represents an alkyl group, a haloalkyl group, or an aryl group which may be substituted, and
D represents a group of the formula: -C(=Y)COX wherein X represents a methoxy group and Y represents a group of the formula: CHOCH₃.

3. Use according to claim 1 or 2, wherein:
A represents an alkyl group which may be substituted with a substituent selected from the group consisting of halogen atoms and alkoxy groups; an alkenyl group or an alkynyl group which may be substituted with a substituent selected from the group consisting of halogen atoms, alkyl groups and alkoxyl groups; an aryl group or a heterocyclic group which may be substituted with a substituent selected from the group consisting of halogen atoms, alkyl groups, haloalkyl groups, alkoxy groups, haloalkoxy groups, alkylthio groups, aryl groups which may be substituted, aryloxy groups which may be substituted and heteroaryloxy groups which may be substituted (the substituents of the aryl groups, aryloxy groups and heteroaryloxy groups are selected from the group consisting of halogen atoms, alkyl groups, haloalkyl groups, alkoxy groups, haloalkoxy groups and alkylthio groups) (in the substituents of the aryl groups and heterocyclic groups, two groups adjacent to each other may together form a ring to be condensed ring with the aryl group or heterocyclic group),
B represents a group of the formula: -OCH₂-, -C≡C-; -CH=CH-; or -CH₂CH₂-,
R¹ represents a hydrogen atom; a halogen atom, an alkyl group; a haloalkyl group; an alkoxy group; a haloalkoxy group; or an aryl group which may be substituted with a substituent selected from the group consisting of halogen atoms, alkyl groups, haloalkyl groups, alkoxy groups and haloalkoxy groups, and
R² represents an alkyl group; a haloalkyl group; or an aryl group which may be substituted with a substituent selected from the group consisting of halogen atoms, alkyl groups, haloalkyl groups, alkoxyl groups and haloalkoxy groups.

4. Use according to any of claims 1 to 3, wherein:
A represents an aryl group or a heterocyclic group which may be substituted with a substituent selected from the group consisting of halogen atoms, alkyl groups, haloalkyl groups, alkoxy groups, haloalkoxy groups and alkylthio groups, aryl groups which may be substituted, aryloxy groups which may be substituted and heteroaryloxy groups which may be substituted) (the substituents of the aryl groups, aryloxy groups and heteroaryloxy groups are selected from the group consisting of halogen atoms, alkyl groups, haloalkyl groups, alkoxy groups, haloalkoxy groups and alkylthio groups), and
B represents a group of the formula: -OCH₂- or -C≡C-.

5. Use of pyrazolylacrylic acid derivative of the following general formula (II): 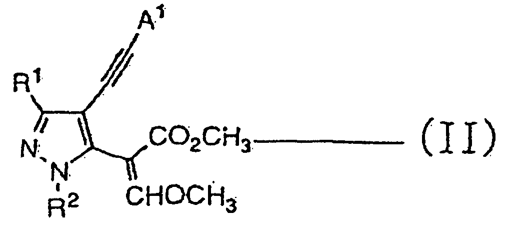 wherein
A¹ represents a phenyl group substituted at, at least, the 4-position or a phenyl group substituted at 3- and 5-positions independently from each other, the substituents being selected from the group consisting of halogen atoms, alkyl groups, haloalkyl groups, alkoxy groups, haloalkoxyl groups, alkylthio groups, aryl groups which may be substituted, aryloxy groups which may be substituted and heteroaryloxy groups which may be substituted (the substituents of the aryl groups, aryloxy groups and heteroaryloxy group are selected from the group consisting of halogen atoms, alkyl groups, haloalkyl groups, alkoxy groups, haloalkoxy groups and alkylthio groups),
R¹ represents a hydrogen atom; a halogen atom; an alkyl group which may be substituted; an alkenyl group which may be substituted; an alkynyl group which may be substituted; an alkoxy group which may be substituted; or an aryl group which may be substituted, and
R² represents a hydrogen atom, an alkyl group; a haloalkyl group; or an aryl group which may be substituted;
with the proviso that the following compounds are excluded: methyl α-{1,3-dimethyl-4-(4-chlorophenylethynyl)-5-pyrazole}-β-methoxyacrylate; methyl α-{1,3-dimethyl-4-(4-fluorophenylethynyl)-5-pyrazole}-β-methoxyacrylate; methyl α-{1,3-dimethyl-4-(4-methoxyphenylethynyl)-5-pyrazole}-β-methoxyacrylate; methyl α-{1,3-dimethyl-4-(4-methylphenylethynyl)-5-pyrazole}-β-methoxyacrylate; methyl α-{1,3-dimethyl-4-(3,4-dichlorophenylethynyl)-5-pyrazole}-β-methoxyacrylate; methyl α-{1,3-dimethyl-4-(3-chloro-4-methoxyphenylethynyl)-5-pyrazole}-β-methoxyacrylate; methyl α-{1,3-dimethyl-4-(2,4-dichlorophenylethynyl)-5-pyrazole}-β-methoxyacrylate; methyl α-{1-methyl-3-trifluoromethyl-4-(4-chlorophenylethynyl)-5-pyrazole}-β-methoxyacrylate; methyl α-{1-methyl-3-trifluoromethyl-4-(4-fluorophenylethynyl)-5-pyrazole}-β-methoxyacrylate; methyl α-{1-methyl-3-trifluoromethyl-4-(3-chloro-4-methoxyphenylethynyl)-5-pyrazole}-β-methoxyacrylate; and methyl α-{1-methyl-3-trifluoromethyl-4-(2,4-dichlorophenylethynyl)-5-pyrazole}-β-methoxyacrylate; as an insecticide or acaricide.

6. Use according to claim 5, wherein:
R¹ represents a hydrogen atom; a halogen atom; an alkyl group; a haloalkyl group; an alkoxy group; a haloalkoxy group; or an aryl group which may be substituted with a substituent selected from the group consisting of halogen atoms, alkyl groups, haloalkyl groups, alkoxy groups and haloalkoxy groups, and
R² represents an alkyl group; a haloalkyl group; or an aryl group which may be substituted with a substituent selected from the group consisting of halogen atoms, alkyl groups, haloalkyl groups, alkoxy groups and haloalkoxy groups.

## Patentansprüche

1. Verwendung eines Pyrazolyl-Derivats der folgenden allgemeinen Formel (I): 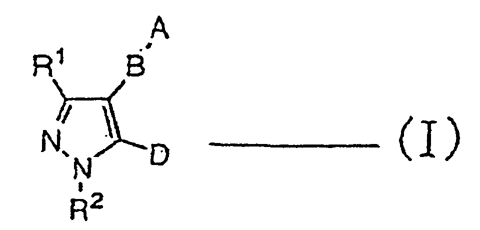 wobei
A ein Wasserstoffatom; einen Alkylrest, der substituiert sein kann; einen Alkenylrest, der substituiert sein kann; einen Alkinylrest, der substituiert sein kann; eine trisubstituierte Silylgruppe, substituiert mit einem Alkylrest und/oder einem Arylrest; einen Arylrest, der substituiert sein kann; oder einen heterocyclischen Rest, der substituiert sein kann, bedeutet;
B eine Einfachbindung; einen Rest der Formel -(G¹)ₙ-G²-(G¹)ₘ-, wobei G¹ ein Sauerstoffatom, ein Schwefelatom, eine Sulfinylgruppe oder eine Sulfonylgruppe bedeutet, G² einen Alkylenrest, einen Alkenylenrest oder einen Alkinylenrest bedeutet und n und m voneinander unabhängig sind und 0 oder 1 bedeuten; eine Carbonylgruppe; einen Rest der Formel -CH₂-O-N=C(R³)-, wobei R³ ein Wasserstoffatom, einen Alkylrest oder einen Halogenalkylrest bedeutet; oder einen Rest der Formel -CH=N-O-(CR³R⁴)ₙ-, wobei R³ und R⁴ jeweils ein Wasserstoffatom, einen Alkylrest oder einen Halogenalkylrest bedeuten; und n 0 oder 1 ist, darstellt;
R¹ ein Wasserstoffatom; ein Halogenatom; einen Alkylrest, der substituiert sein kann; einen Alkenylrest, der substituiert sein kann; einen Alkinylrest, der substituiert sein kann; einen Alkoxyrest, der substituiert sein kann; oder einen Arylrest, der substituiert sein kann; bedeutet;
R² ein Wasserstoffatom; einen Alkylrest; einen Halogenalkylrest; oder einen Arylrest, der substituiert sein kann, bedeutet; und
D einen Rest der Formel -C(=Y)COX, wobei X eine Hydroxylgruppe, einen Alkoxyrest oder eine Alkylaminogruppe bedeutet, Y einen Rest der Formel CH-(G³)ₙ-G⁴, wobei G³ ein Sauerstoffatom oder ein Schwefelatom bedeutet; G⁴ einen Alkylrest oder einen Halogenalkylrest bedeutet, und n 0 oder 1 bedeutet; einen Rest der Formel N-O-G⁴, wobei G⁴ einen Alkylrest oder einen Halogenalkylrest bedeutet; oder einen Rest der Formel -N(R⁵)CO₂G⁵, wobei R⁵ einen Alkylrest, einen Alkenylrest, einen Alkinylrest, einen Alkylthioalkylrest oder einen Alkoxyalkylrest bedeutet und G⁵ einen Alkylrest bedeutet; darstellt;
als Insektizid oder Akarizid.

2. Verwendung nach Anspruch 1, wobei:
B einen Rest der Formel -OCH₂-; -CH₂-O-; -C≡C-; -CH=CH-; -CH₂CH₂₇-; einen Rest der Formel -CH₂ON=C(R³)-, wobei R³ ein Wasserstoffatom, einen Alkylrest oder einen Halogenalkylrest bedeutet; oder einen Rest der Formel -CH=NO-(CR³R⁴)ₙ-, wobei R³ und R⁴ jeweils ein Wasserstoffatom, einen Alkylrest oder einen Halogenalkylrest bedeuten; und n 0 oder 1 ist; darstellt;
R¹ ein Wasserstoffatom, ein Halogenatom, einen Alkylrest, einen Halogenalkylrest, einen Alkoxyrest, einen Halogenalkoxyrest oder einen Arylrest, der substituiert sein kann, bedeutet;
R² einen Alkylrest, einen Halogenalkylrest oder einen Arylrest, der substituiert sein kann, bedeutet; und
D einen Rest der Formel -C(=Y)COX, wobei X eine Methoxygruppe bedeutet und Y einen Rest der Formel CHOCH₃ bedeutet, darstellt.

3. Verwendung nach Anspruch 1 oder 2, wobei
A einen Alkylrest, der mit einem Substituenten, ausgewählt aus Halogenatomen und Alkoxyresten, substituiert sein kann; einen Alkenylrest oder einen Alkinylrest, der mit einem Substituenten, ausgewählt aus Halogenatomen, Alkylresten und Alkoxyresten, substituiert sein kann; einen Arylrest oder einen heterocyclischen Rest, der mit einem Substituenten, ausgewählt aus Halogenatomen, Alkylresten, Halogenalkylresten, Alkoxyresten, Halogenalkoxyresten, Alkylthiogruppen, Arylresten, die substituiert sein können, Aryloxyresten, die substituiert sein können, und Heteroaryloxyresten, die substituiert sein können, substituiert sein kann, darstellt (die Substituenten der Arylreste, Aryloxyreste und Heteroaryloxyreste sind ausgewählt aus Halogenatomen, Alkylresten, Halogenalkylresten, Alkoxyresten, Halogenalkoxyresten und Alkylthiogruppen) (bei den Substituenten der Arylreste und heterocyclischen Reste können zwei zueinander benachbarte Reste zusammen einen Ring bilden, um einen kondensierten Ring mit dem Arylrest oder heterocyclischen Rest zu ergeben);
B einen Rest der Formel -OCH₂-, -C≡C-; -CH=CH-; oder -CH₂CH₂- bedeutet;
R¹ ein Wasserstoffatom; ein Halogenatom, einen Alkylrest; einen Halogenalkylrest; einen Alkoxyrest; einen Halogenalkoxyrest; oder einen Arylrest, der mit einem Substituenten, ausgewählt aus Halogenatomen, Alkylresten, Halogenalkylresten, Alkoxyresten und Halogenalkoxyresten, substituiert sein kann, bedeutet; und
R² einen Alkylrest; einen Halogenalkylrest; oder einen Arylrest, der mit einem Substituenten, ausgewählt aus Halogenatomen, Alkylresten, Halogenalkylresten, Alkoxyresten und Halogenalkoxyresten, substituiert sein kann, bedeutet.

4. Verwendung nach einem der Ansprüche 1 bis 3, wobei:
A einen Arylrest oder einen heterocyclischen Rest, der mit einem Substituenten, ausgewählt aus Halogenatomen, Alkylresten, Halogenalkylresten, Alkoxyresten, Halogenalkoxyresten und Alkylthiogruppen, Arylresten, die substituiert sein können, Aryloxyresten, die substituiert sein können, und Heteroaryloxyresten, die substituiert sein können, substituiert sein kann, bedeutet (die Substituenten der Arylreste, Aryloxyreste und Heteroaryloxyreste sind ausgewählt aus Halogenatomen, Alkylresten, Halogenalkylresten, Alkoxyresten, Halogenalkoxyresten und Alkylthiogruppen); und
B einen Rest der Formel -OCH₂- oder -C=C- bedeutet.

5. Verwendung eines Pyrazolylacrylsäure-Derivats der folgenden allgemeinen Formel (II): 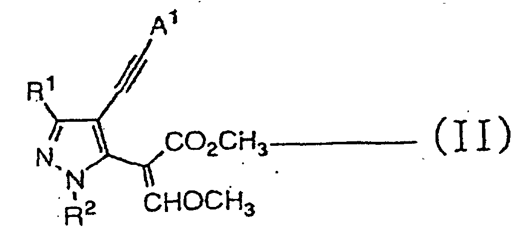 wobei
A¹ eine Phenylgruppe, die zumindest an der Position 4 substituiert ist, oder eine Phenylgruppe, die an den Positionen 3 und 5 unabhängig voneinander substituiert ist, bedeutet, wobei die Substituenten ausgewählt sind aus Halogenatomen, Alkylresten, Halogenalkylresten, Alkoxyresten, Halogenalkoxyresten, Alkylthiogruppen, Arylresten, die substituiert sein können, Aryloxyresten, die substituiert sein können, und Heteroaryloxyresten, die substituiert sein können (die Substituenten der Arylreste, Aryloxyreste und Heteroaryloxyreste sind ausgewählt aus Halogenatomen, Alkylresten, Halogenalkylresten, Alkoxyresten, Halogenalkoxyresten und Alkylthiogruppen);
R¹ ein Wasserstoffatom; ein Halogenatom; einen Alkylrest, der substituiert sein kann; einen Alkenylrest, der substituiert sein kann; einen Alkinylrest, der substituiert sein kann; einen Alkoxyrest, der substituiert sein kann; oder einen Arylrest, der substituiert sein kann, bedeutet; und
R² ein Wasserstoffatom; einen Alkylrest; einen Halogenalkylrest; oder einen Arylrest, der substituiert sein kann, bedeutet;
mit der Maßgabe, daß die folgenden Verbindungen ausgenommen sind: Methyl-α-{1,3-dimethyl-4-(4-chlorphenylethinyl)-5-pyrazol}-β-methoxyacrylat; Methyl-α-{1,3-dimethyl-4-(4-fluorphenylethinyl)-5-pyrazol}-β-methoxyacrylat; Methyl-α-{1,3-dimethyl-4-(4-methoxyphenylethinyl)-5-pyrazol}-β-methoxyacrylat; Methyl-α-{1,3-dimethyl-4-(4-methylphenylethinyl)-5-pyrazol}-β-methoxyacrylat; Methyl-α-{1,3-dimethyl-4-(3,4-dichlorphenylethinyl)-5-pyrazol}-β-methoxyacrylat; Methyl-α-{1,3-dimethyl-4-(3-chlor-4-methoxyphenylethinyl)-5-pyrazol}-β-methoxyacrylat; Methyl-α-{1,3-dimethyl-4-(2,4-dichlorphenylethinyl)-5-pyrazol}-β-methoxyacrylat; Methyl-α-{1-methyl-3-trifluormethyl-4-(4-chlorphenylethinyl)-5-pyrazol}-β-methoxyacrylat; Methyl-α-{1-methyl-3-trifluormethyl-4-(4-fluorphenylethinyl)-5-pyrazol}-β-methoxyacrylat; Methyl-α-{1-methyl-3-trifluormethyl-4-(3-chlor-4-methoxyphenylethinyl)-5-pyrazol}-β-methoxyacrylat; und Methyl-a-{1-methyl-3-trifluormethyl-4-(2,4-dichlorphenylethinyl)-5-pyrazol}-β-methoxyacrylat als Insektizid oder Akarizid.

6. Verwendung nach Anspruch 5, wobei:
R¹ ein Wasserstoffatom; ein Halogenatom; einen Alkylrest; einen Halogenalkylrest; einen Alkoxyrest; einen Halogenalkoxyrest; oder einen Arylrest, der mit einem Substituenten, ausgewählt aus Halogenatomen, Alkylresten, Halogenalkylresten, Alkoxyresten und Halogenalkoxyresten, substituiert sein kann, bedeutet; und
R² einen Alkylrest; einen Halogenalkylrest; oder einen Arylrest, der mit einem Substituenten, ausgewählt aus Halogenatomen, Alkylresten, Halogenalkylresten, Alkoxyresten und Halogenalkoxyresten, substituiert sein kann, bedeutet.

## Revendications

1. Utilisation d'un dérivé pyrazolyle de formule générale suivante (I) : 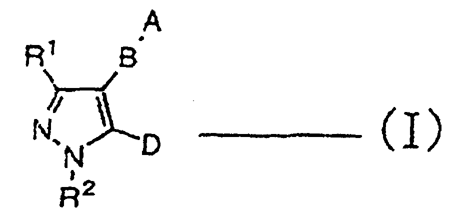 dans laquelle
A représente un atome d'hydrogène ; un groupe alkyle qui peut être substitué ; un groupe alcényle qui peut être substitué ; un groupe alcynyle qui peut être substitué ; un groupe silyle tri-substitué substitué avec un groupe alkyle et/ou un groupe aryle ; un groupe aryle qui peut être substitué ; ou un groupe hétérocyclique qui peut être substitué ;
B représente une liaison simple ; un groupe de formule : -(G¹)ₙ-G²-(G¹)ₘ- dans laquelle G' représente un atome d'oxygène, un atome de soufre, un groupe sulfinyle ou un groupe sulfonyle, G² représente un groupe alkylène, un groupe alcénylène ou un groupe alcynylène, et n et m sont indépendants l'un de l'autre et représentent 0 ou 1 ; un groupe carbonyle ; un groupe de formule : -CH₂-O-N=C(R³)- dans laquelle R³ représente un atome d'hydrogène, un groupe alkyle ou un groupe halogénoalkyle ; ou un groupe de formule : -CH=N-O-(CR³R⁴)ₙ- dans laquelle R³ et R⁴ représentent chacun un atome d'hydrogène, un groupe alkyle ou un groupe halogénoalkyle ; et n est 0 ou 1,
R' représente un atome d'hydrogène ; un atome d'halogène, un groupe alkyle qui peut être substitué ; un groupe alcényle qui peut être substitué ; un groupe alcynyle qui peut être substitué ; un groupe alcoxy qui peut être substitué ; ou un groupe aryle qui peut être substitué,
R² représente un atome d'hydrogène ; un groupe alkyle ; un groupe halogénoalkyle ; ou un groupe aryle qui peut être substitué, et
D représente un groupe de formule : -C(=Y)COX dans laquelle X représente un groupe hydroxyle, un groupe alcoxy ou un groupe alkylamino, Y représente un groupe de formule: CH-(G³)ₙ-G⁴ dans laquelle G³ représente un atome d'oxygène ou un atome de soufre, G⁴ représente un groupe alkyle ou un groupe halogénoalkyle, et n représente 0 ou 1, un groupe de formule : N-O-G⁴ dans laquelle G⁴ représente un groupe alkyle ou un groupe halogénoalkyle; ou un groupe de formule : -N(R⁵)CO₂G⁵ dans laquelle R⁵ représente un groupe alkyle, un groupe alcényle, un groupe alcynyle, un groupe alkylthioalkyle ou un groupe alcoxyalkyle, et G⁵ représente un groupe alkyle ; comme insecticide ou acaricide.

2. Utilisation selon la revendication 1, dans laquelle :
B représente un groupe de formule -OCH₂-, -CH₂-O-, -C≡C- ; -CH=CH- ; -CH₂CH₂- ; un groupe de formule : -CH₂ON=C(R³)- dans laquelle R³ représente un atome d'hydrogène, un groupe alkyle ou halogénoalkyle ; ou un groupe de formule :
-CH=NO-(CR³R⁴)ₙ- dans laquelle R³ et R⁴ représentent chacun un atome d'hydrogène, un groupe alkyle ou un groupe halogénoalkyle ; et n est 0 ou 1,
R' représente un atome d'hydrogène, un atome d'halogène, un groupe alkyle, un groupe halogénoalkyle, un groupe alcoxy, un groupe halogénoalcoxy, ou un groupe aryle qui peut être substitué,
R² représente un groupe alkyle, un groupe halogénoalkyle, ou un groupe aryle qui peut être substitué, et
D représente un groupe de formule : -C(=Y)COX dans laquelle X représente un groupe méthoxy et Y représente un groupe de formule : CHOCH₃.

3. Utilisation selon la revendication 1 ou 2, dans laquelle :
A représente un groupe alkyle qui peut être substitué avec un substituant choisi dans le groupe constitué des atomes d'halogène et des groupes alcoxy ; un groupe alcényle ou un groupe alcynyle qui peut être substitué avec un substituant choisi dans le groupe constitué des atomes d'halogène, des groupes alkyle et des groupes alcoxy ; un groupe aryle ou un groupe hétérocyclique qui peut être substitué avec un substituant choisi dans le groupe constitué des atomes d'halogène, des groupes alkyle, des groupes halogénoalkyle, des groupes alcoxy, des groupes halogénoalcoxy, des groupes alkylthio, des groupes aryle qui peuvent être substitués, des groupes aryloxy qui peuvent être substitués et des groupes hétéroaryloxy qui peuvent être substitués (les substituants des groupes aryle, des groupes aryloxy et des groupes hétéroaryloxy sont choisis dans le groupe constitué des atomes d'halogène, des groupes alkyle, des groupes halogénoalkyle, des groupes alcoxy, des groupes halogénoalcoxy et des groupes alkylthio) (dans les substituants des groupes aryle et des groupes hétérocycliques, deux groupes adjacents l'un par rapport à l'autre peuvent former ensemble un cycle à condenser avec le groupe aryle ou le groupe hétérocyclique),
B représente un groupe de formule : -OCH₂-, -C≡C- ; -CH=CH- ; ou -CH₂CH₂-,
R' représente un atome d'hydrogène ; un atome d'halogène, un groupe alkyle ; un groupe halogénoalkyle ; un groupe alcoxy ; un groupe halogénoalcoxy ; ou un groupe aryle qui peut être substitué avec un substituant choisi dans le groupe constitué des atomes d'halogène, des groupes alkyle, des groupes halogénoalkyle, des groupes alcoxy et des groupes halogénoalcoxy ; et
R² représente un groupe alkyle ; un groupe halogénoalkyle ; ou un groupe aryle qui peut être substitué avec un substituant choisi dans le groupe constitué des atomes d'halogène, des groupes alkyle, des groupes halogénoalkyle, des groupes alcoxy et des groupes halogénoalcoxy.

4. Utilisation selon l'une quelconque des revendications 1 à 3, dans laquelle :
A représente un groupe aryle ou un groupe hétérocyclique qui peut être substitué avec un substituant choisi dans le groupe constitué des atomes d'halogène, des groupes alkyle, des groupes halogénoalkyle, des groupes alcoxy, des groupes halogénoalcoxy et des groupes alkylthio, des groupes aryle qui peuvent être substitués, des groupes aryloxy qui peuvent être substitués et des groupes hétéroaryloxy qui peuvent être substitués (les substituants des groupes aryle, des groupes aryloxy et des groupes hétéroaryloxy sont choisis dans le groupe constitué des atomes d'halogène, des groupes alkyle, des groupes halogénoalkyle, des groupes alcoxy, des groupes halogénoalcoxy et des groupes alkylthio), et
B représente un groupe de formule : -OCH₂- ou -C≡C-.

5. Utilisation d'un dérivé d'acide pyrazolylacrylique de formule générale suivante (II) : 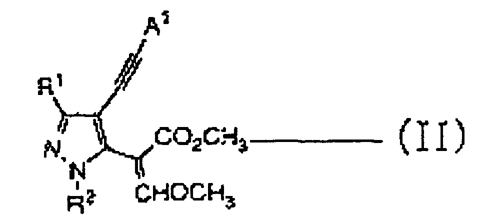 dans laquelle
A¹ représente un groupe phényle substitué, au moins en position 4, ou un groupe phényle substitué en positions 3 et 5 indépendamment l'une de l'autre, les substituants étant choisis dans le groupe constitué des atomes d'halogène, des groupes alkyle, des groupes halogénoalkyle, des groupes alcoxy, des groupes halogénoalcoxy, des groupes alkylthio, des groupes aryle qui peuvent être substitués, des groupes aryloxy qui peuvent être substitués et des groupes hétéroaryloxy qui peuvent être substitués (les substituants des groupes aryle, des groupes aryloxy et des groupes hétéroaryloxy sont choisis dans le groupe constitué des atomes d'halogène, des groupes alkyle, des groupes halogénoalkyle, des groupes alcoxy, des groupes halogénoalcoxy et des groupes alkylthio),
R¹ représente un atome d'hydrogène ; un atome d'halogène, un groupe alkyle qui peut être substitué ; un groupe alcényle qui peut être substitué, un groupe alcynyle qui peut être substitué ; un groupe alcoxy qui peut être substitué ; ou un groupe aryle qui peut être substitué ; et
R² représente un atome d'hydrogène, un groupe alkyle ; un groupe halogénoalkyle ; ou un groupe aryle qui peut être substitué ;
avec la condition que les composés suivants soient exclus : α-{1,3-diméthyl-4-(4-chloro-phényléthynyl)-5-pyrazole}-β-méthoxyacrylate de méthyle; α-{1,3-diméthyl-4-(4-fluorophényléthynyl)-5-pyrazole}-β-méthoxyacrylate de méthyle ; α-{1,3-diméthyl-4-(4-méthoxyphényléthynyl)-5-pyrazole}-β-méthoxyacrylate de méthyle; α-{1,3-diméthyl-4-(4-méthylphényléthynyl)-5-pyrazole}-β-méthoxyacrylate de méthyle ; α-{1,3-diméthyl-4-(3,4-dichlorophényléthynyl)-5-pyrazole}-β-méthoxyacrylate de méthyle; α-{1,3-diméthyl-4-(3-chloro-4-méthoxyphényléthynyl)-5-pyrazole}-β-méthoxyacrylate de méthyle; α-{1,3-diméthyl-4-(2,4-dichlorophényléthynyl)-5-pyrazole}-β-méthoxyacrylate de méthyle; α-{1-méthyl-3-trifluorométhyl-4-(4-chlorophényléthynyl)-5-pyrazole}-β-méthoxyacrylate de méthyle ; α-{1-méthyl-3-trifluorométhyl-4-(4-fluorophényléthynyl)-5-pyrazole}-β-méthoxyacrylate de méthyle ; α-{1-méthyl-3-trifluorométhyl-4-(3-chloro-4-méthoxyphényléthynyl)-5-pyrazole}-β-méthoxyacrylate de méthyle; et α-{1-méthyl-3-trifluorométhyl-4-(2,4-dichlorophényléthynyl)-5-pyrazole}-β-méthoxyacrylate de méthyle; comme insecticide ou acaricide.

6. Utilisation selon la revendication 5, dans laquelle :
R' représente un atome d'hydrogène ; un atome d'halogène ; un groupe alkyle ; un groupe halogénoalkyle ; un groupe alcoxy ; un groupe halogénoalcoxy ; ou un groupe aryle qui peut être substitué avec un substituant choisi dans le groupe constitué des atomes d'halogène, des groupes alkyle, des groupes halogénoalkyle, des groupes alcoxy et des groupes halogénoalcoxy ; et
R² représente un groupe alkyle ; un groupe halogénoalkyle ; ou un groupe aryle qui peut être substitué avec un substituant choisi dans le groupe constitué des atomes d'halogène, des groupes alkyle, des groupes halogénoalkyle, des groupes alcoxy et des groupes halogénoalcoxy.
